(19) Europäisches Patentamt
European Patent Office
Office européen des brevets

(11) EP 0 345 593 B2

(12) **NEW EUROPEAN PATENT SPECIFICATION**

(45) Date of publication and mention of the opposition decision:
**04.02.1998 Bulletin 1998/06**

(45) Mention of the grant of the patent:
**18.08.1993 Bulletin 1993/33**

(21) Application number: **89109679.4**

(22) Date of filing: **30.05.1989**

(51) Int. Cl.[6]: **C07D 307/79**, C07D 307/92, A61K 31/34

# (54) 2-substituted coumaran derivatives

In 2-Stellung substituierte Cumaranderivate

Dérivés du coumarane substitués en position 2

(84) Designated Contracting States:
**AT BE CH DE ES FR GB GR IT LI LU NL SE**

(30) Priority: **10.06.1988 JP 143859/88**

(43) Date of publication of application:
**13.12.1989 Bulletin 1989/50**

(73) Proprietor:
**Takeda Chemical Industries, Ltd.**
**Chuo-ku, Osaka (JP)**

(72) Inventors:
- **Goto, Giichi**
  **Toyono-gun Osaka 563-01 (JP)**
- **Ohkawa, Shigenori**
  **Takatsuki Osaka 569 (JP)**
- **Fukuda, Naohisa**
  **Kawanishi Hyogo 666-01 (JP)**

(74) Representative:
**Lederer, Franz, Dr. et al**
**Lederer, Keller & Riederer**
**Patentanwälte**
**Prinzregentenstrasse 16**
**80538 München (DE)**

(56) References cited:
- **CHEMICAL ABSTRACTS, vol. 108, no. 10, 07 March 1988, Columbus, OH (US); p. 445, no. 82107a#**
- **CHEMICAL ABSTRACTS, vol. 110, 1989, 27 February 1989, Columbus, OH (US); p. 630, no. 75294x#**
- **CHEMICAL ABSTRACTS, vol. 105, no. 23, 08 December 1986, Columbus, OH (US); p. 510, no. 207914h#**
- **CHEMICAL AND PHARMACEUTICAL BULLETIN, vol. 30, no. 8, August 1982, K. OKAMOTO et al., pp. 2797-2819#**
- **JOURNAL OF ORGANIC CHEMISTRY, vol. 54, no. 3, 03 February 1989, American Chemical Society, Easton (US); S. BROWNSTEIN et al., pp. 560-569#**
- **Chemical Abstracts, vol. 108, 1988, no. 48924e**
- **Chem. Pharm. Bull., 1986, vol. 34, pp. 3960-3963**
- **J. Am. Chem. Soc., 1985, vol. 107, pp. 7053-7065**
- **Prostaglandins 1981, vol. 22. p. 255**
- **FEBS Lett., 1986, vol. 205, pp. 117-120**
- **Helv. Chim. Acta, 1963, vol. 46, pp. 650-671**
- **Helv. Chim. Acta, 1976, vol. 59, pp. 290-306**
- **J. Heterocycl. Chem., 1991, vol. 28, pp. 1395-1403**
- **Advances in Inflammation Research, vol. 12, 1988, pp. 159-172**
- **J. Am. Chem. Soc., vol. 65, 1943, pp. 1594-1599**
- **J. Am. Chem. Soc., vol. 63, 1941, pp. 1887-1890**
- **J. Am. Chem. Soc., vol. 70, 1948, pp. 2690-2695**
- **Helv. Chim. Acta, vol. 31, 1948, pp. 1505-1513**
- **J. Am. Chem. Soc., vol. 105, 1983, pp. 5950-5951**

Remarks:
The file contains technical information submitted after the application was filed and not included in this specification

EP 0 345 593 B2

## Description

This invention relates to 2-substituted coumaran derivatives.

While several types of compounds of coumaran derivatives have been synthesized [Journal of American Chemical Society (J. Am. Chem. Soc.) 105, 5950(1983); ibid, 107, 7053(1985)], substantially no reports have been made on their pharmacological actions. The vitamin E analog (I) disclosed in Chemical Abstracts, 1986, 105:207914h contains 16 carbon atoms in the side chain and is therefore outside of the group of 2,3-dihydrobenzofuran derivatives defined in present Claim 1 wherein the number of carbon atoms in the side chain is limited to 15.

Based on the disclaimer in Claim 1 (in position alpha to the benzofuran ring) the compound 2-(2-(2,3-dihydro-5-hydroxy-4,6,7-trimethylbenzofuranyl))-propionic acid (XVb) disclosed in Chemical and Pharmaceutical Bulletin, 1982, pages 2797-2819 is excluded from present Claim 1.

Chemical Abstracts, 1988, 108:82107a discloses 3,4-dihydro-2H-benzo**pyran** derivatives and Chemical Abstracts, 1989, 110:75294x 2,3-dihydrobenzofurans having a side-chain $-(CH_2)_nOR^2$ which is none of the options given for $R^2$ in the present claims.

The present inventors synthesized various types of coumaran derivatives and found that they had inhibitory actions on 5-lipoxygenase and 12-lipoxygenase participating in the biosynthesis of leukotrienes and lipoxins, and they have continued the research work diligently to accomplish the present invention.

The present invention is to provide a compound of the formula:

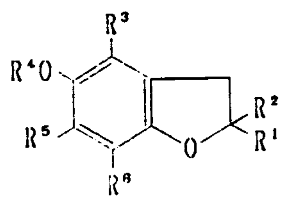

(I)

wherein $R^1$ is hydrogen or $C_{1-6}$ alkyl; $R^2$ is methyl which is substituted by carboxy, $c_{2-5}$ alkoxycarbonyl, cyano, halogen, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with one or more substituents selected from $c_{1-20}$ alkyl, $C_{1-6}$ alkyl substituted by hydroxyl, carboxyl, $c_{2-5}$ alkoxycarbonyl, piperazyl or phenylthio, $C_{2-4}$ alkenyl, halogen, nitro, formyl, $C_{1-3}$ alkoxy, carboxy, trifluoromethyl, di-$C_{1-3}$-alkylamino, $C_{5-7}$ cycloalkyl and $C_{1-3}$ alkylthio; or $R^2$ is a $C_{2-15}$ alkyl or $C_{2-15}$ alkenyl group which may be substituted by carboxy, $C_{2-5}$ alkoxycarbonyl, halogen, cyano, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, chain-like $c_{1-10}$ alkanoyl or cyclic $c_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $C_{1-3}$ alkoxy, hydroxyl, nitro and halogen, or a salt thereof, with the exclusion of 5-hydroxy-4,6,7-trimethyl-2-isopropyl-2,3-dihydrobenzofuran, 5-acetoxy-4,6,7-trimethyl-2-isopropyl-2,3-dihydrobenzofuran, 5-hydroxy-4,6,7-trimethyl-2-n-propyl-2,3-dihydrobenzofuran, 5-hydroxy-4,6,7-trimethyl-2-ethyl-2,3-dihydrobenzofuran, 5-hydroxy-4,6,7-trimethyl-2-n-tridecyl-2,3-dihydrobenzofuran, and 5-hydroxy-2,4,6,7-tetramethyl-2-(3',7',11'-trimethyl-dodecyl)-2,3-dihydrobenzofuran.

The present invention is also to provide a compound of the formula (I) as defined above with the exclusion of 5-hydroxy-2,4,6,7-tetramethyl-2-(3',7',11'-trimethyl-dodecyl)-2,3-dihydrobenzofuran, as a medicament for the treatment of the human or animal body by therapy.

The present invention is further to provide the use of a compound of the formula (I) as defined above, for the preparation of a medicament for ameliorating the circulatory system, for treating the central nervous system or for treating allergic diseases.

Referring to compounds represented by the above-mentioned (I), the term $C_{1-6}$ alkyl represented by $R^1$ is exemplified by radicals such as methyl, ethyl, propyl, i-propyl, butyl, i-butyl, sec-butyl, t-butyl, amyl, hexyl, etc., especially $C_{1-3}$ alkyl (methyl, ethyl, propyl, i-propyl) being preferable.

As substituents of the substituted methyl represented by $R^2$, mention is made of phenyl, 1-naphthyl, 2-naphthyl, indanyl, tetralyl, or a heterocyclic group selected from 2-thienyl, 3-thienyl, 2-pyridyl, 3-pyridyl, 4-pyridyl, 1-imidazolyl, 5-thiazolyl morpholino, thiomorpholino; halogen such as fluorine, chlorine, bromine, iodine; carboxyl, $C_{2-5}$-alkoxycarbonyl such as methoxycarbonyl, ethoxycarbonyl, ; cyano.

Further, the phenyl and the heterocyclic groups may have one to two or more substituents at an optional position of the ring. As the said substituents, mention is made of unsubstituted $C_{1-20}$ alkyl (e.g. methyl, ethyl, propyl, isopropyl, butyl, t-butyl, pentyl, hexyl, octyl, nonyl, decyl, undecyl, dodecyl, tridecyl, tetradecyl, pentadecyl, hexadecyl, heptadecyl, octadecyl, nonadecyl, eicosyl) ; ($C_{1-6}$) alkyl optionally substituted with hydroxyl group, carboxyl, $C_{2-5}$ alkoxycarbonyl, piperazyl, or phenylthio, $C_{2-4}$ alkenyl (e.g vinyl), $C_{2-5}$ alkoxycarbonyl such as methoxycarbonyl, ethoxycarbonyl, etc. , hydroxyl group, halogen, such as fluorine, chlorine, bromine; nitro, formyl, $C_{1-3}$ alkoxy (methoxy, etc.), carboxyl, trifluoromethyl, di-$C_{1-3}$ alkylamino, $C_{5-7}$ cycloalkyl or $C_{1-3}$ alkylthio.

If $R^2$ is a $C_{2-15}$ alkyl or $C_{2-15}$ alkenyl group which is unsubstituted at the carbon atom in position alpha to the benzofuran ring, mention is made of straight-chain or branched groups, and when it is alkenyl, the number of double bonds is usually 1 to 5, and these double bonds may be conjugated.

As the above-mentioned alkyl or alkenyl groups, those having 2 to 6 carbon atoms are preferable, as exemplified ethyl, i-propyl, butyl, i-butyl, sec-butyl, t-butyl, pentyl, hexyl, 1-propenyl, 2-butenyl, 1,3-butadienyl, 2-pentenyl, vinyl, 2-propenyl, isopropenyl.

Referring to the substituents of $C_{2-15}$ alkyl or alkenyl groups, represented by $R^2$ mention is made of $C_{2-5}$ alkoxycarbonyl, such as methoxycarbonyl, ethyoxycarbonyl, propoxycarbonyl, i-propoxycarboxyl and, as halogen, mention is made of fluorine, bromine, chlorine and iodine. As the phenyl and heterocyclic groups, mention is made of the respectively those similar to the groups described as above, and, when these groups have further substituents are mentioned those similar to the groups described as above.

$R^3$ is $C_{1-6}$ alkyl such as methyl, ethyl, propyl, i-propyl, butyl, i-butyl, sec-butyl, t-bulyl, amyl, hexyl, especially, $C_{1-3}$ alkyls (methyl, ethyl, propyl, i-propyl,) are preferable.

$R^4$ means ($C_{1-10}$) alkanoyl or cyclic ($C_{3-10}$) alkanoyl, such as formyl, acetyl, propionyl, isobutyryl, decanoyl, cyclopentyl- or cyclohexylcarbonyl.

$R^5$ and $R^6$ as $C_{1-6}$ alkyl each are exemplified by methyl, ethyl, propyl, i-propyl, butyl, i-butyl, sec-butyl, t-butyl, amyl, hexyl, especially preferable ones being $C_{1-3}$alkyl (methyl, ethyl, propyl, i-propyl, $R^5$ and $R^6$ in the meaning $C_{1-3}$ alkoxy are exemplified by methoxy, ethoxy, propoxy, i-propoxy.

When $R^5$ and $R^6$ combined represent butadienylene, as the substituents on thus-formed condensed benzene ring are mentioned one to three ($C_{1-3}$) alkyls, ($C_{1-3}$) alkoxy (methoxy, ethoxy, propoxy,), hydroxyl group, nitro and halogen.

The compound (I) may, in accordance with the kinds of substituents thereon, form corresponding salts, and the salts are exemplified by those with an organic acid (e.g. acetic acid, propionic acid, oxalic acid, maleic acid ) or an inorganic acid (e.g. hydrochloric acid, sulfuric acid, phosphoric acid), or those with a base such as an alkali metal (potassium, sodium), an alkaline earth metal (calcium, magnesium), ammonia,, and, among them, physiologically acceptable ones are especially preferable.

The compound (I) can be produced by, for example, by allowing a compound represented by the formula

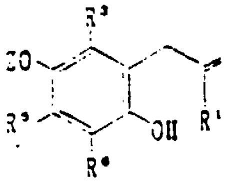

(II)

wherein $R^1$, $R^3$, $R^5$ and $R^6$ are of the same meaning defined as above, Z is hydrogen or a hydroxyl-protecting group, to react with halogen molecule in the presence of a base to cause ring-closure, or by subjecting the compound (II) to the treatment with a peracid in the presence of a base to cause ring closure, followed by allowing thus ring-closed compound, in case of the 2-hydroxymethyl derivative, to react with an oxidizing agent, then subjecting thus-obtained compound to addition-elimination reaction with a compound represented by the formula

$$(C_6H_5)_3P^{\oplus}\text{-}R^{2'}X^{\ominus} \quad \text{(III)}$$

wherein X is halogen, $R^{2'}$ is a hydrocarbon residue whose carbon number is less by one than that of $R^2$, followed by, when desired, subjecting the resultant to deprotection, acylation, hydrogenation or(and) substituent-exchange reaction, respectively.

As the hydroxyl-protecting group, $C_{2-4}$ alkanoyl such as acetyl, propionyl, is mentioned.

The ring-closure reaction with the aid of halogen is carried out by allowing, for example, bromine to react in an organic solvent such as halogenated carbon (e.g. chloroform, methylene chloride,) or acetic acid, at temperatures ranging from -5°C to 80°C.

And, the cyanation can be carried out, in general, by allowing, for example, sodium cyanide or potassium cyanide to react in a solvent such as dimethyl sulfoxide, dimethyl formamide, etc. at temperatures ranging from 60°C to 100°C for 1 to 24 hours. In this case, the protecting group is hydrolyzed with a small volume of water present in the reaction system to give a 5-hydroxy compound at one stroke.

The ring-closure reaction by the use of a peracid is conducted by using a peracid such as m-chloroperbenzoic acid in an organic solvent such as methylene chloride in the presence of a base such as triethylamine at temperatures ranging from -10°C to 50°C. And, the oxidation is conducted by using an oxidizing agent obtained from dimethyl sulfoxide and oxalyl chloride, chromium trioxide, in an organic solvent such as methylene chloride, acetone, and, when desired, in the presence of a base such as triethylamine at temperatures ranging from -78°C to 25°C.

The addition-elimination reaction (Wittig reaction) is conducted by using, as the base, sodium hydride, sodium hydrobromide, sodium alcoholate, n-butyl lithium, lithium diisopropyl amide, etc., in a solvent such as dimethyl sulfoxide, tetrahydrofuran, dimethoxy ethane, at temperatures ranging from -78°C to 80°C for about 0.5 to 24 hours.

And, when the double bond is hydrogenated, the object compound can be obtained in accordance with a conventional method using a catalyst such as palladium-carbon, etc.

The elimination (hydrolysis) of the hydroxy-protecting group can be conducted under the conditions of conventional ester hydrolysis, but, when the product is unstable against oxygen under basic conditions, the reaction is conducted under argon atmosphere to thereby obtain the desired hydrolyzate in a good yield.

The acylation is carried out by using a desired acylating agent (acid anhydride, acid halogenide), when necessary, in the presence of a basic catalyst (preferably sodium hydride, potassium carbonate, pyridine and triethylamine) or an acid catalyst (sulfuric acid, hydrogen chloride), in an organic solvent (e.g. dimethylformamide, acetone, tetrahydrofuran) at temperatures ranging from about -10°C to 100°C for about 10 minutes to 15 hours.

Thus-obtained compound (I) can be isolated by conventional separation purification means (extraction, chromatography, recrystallization).

And, when the compound (I) exists in the state of diastereomer, the respective components can be isolated by the above-mentioned separation purification means.

And, when the compound (I) is an optically active compound, it can be resolved into d-compound and ℓ-compound by conventional means for optical resolution.

The starting compound (II) can be synthesized by, for example, the method described below. More specifically, the monoacetate (IV) of hydroquinone is allowed to react with aryl halogenide in the presence of a base to lead to allyl ether (V), followed by subjecting (V) to Claisen rearrangement to give (II).

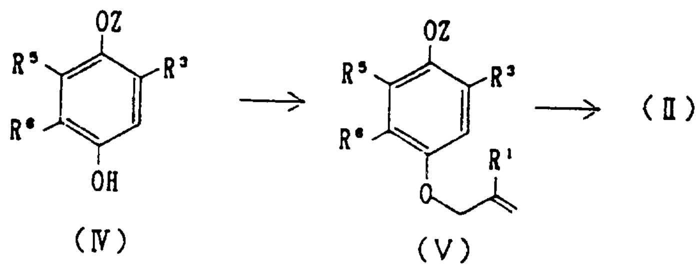

The compound (I) of this invention has an action of inhibiting production of 5-lipoxygenase-type metabolite [leucotrienes, 5-hydroperoxyeicosatetraenoic acid (HPETE), 5-hydroxyeicosatetraenoic acid (HETE), lipoxins, leucotoxins, etc.] and 12-lipoxygenase-type metabolite (12-HPETE, 12-HETE), and, therefore, the compound (I) can be used advantageously as an agent for ameliorating circulatory system, an anti-allergic agent, an agent acting on central nervous system.

The compound (I) can be safely administered, orally or non-orally singly or as a pharmaceutical composition prepared by mixing the compound (I) with a per se known pharmaceutically acceptable carrier, excipient, etc. (e.g. tablet, capsule, liquid, injection, suppository), to mammals (rat, horse, cow, monkey, human). While the dosage varies with subjects of administration, administration routes, symptoms., in the case of, for example, administering orally to an adult patient suffering from diseases of circulatory system, it is convenient to administer with about 0.1 mg/kg to 20 mg/kg/body weight/dose, preferably 0.2 mg/kg to 10 mg/kg/body weight, once to three times a day.

Experimental Example 1:5-Lypoxygenase Inhibiting Action

In 0.5 mℓ of MCM (mast cell medium) was suspended $10^7$ of rat basophilic leukemia (RBL-1) cells. To this suspension was added the test solution previously prepared [consisting of 0.5 mℓ of MCM, 50 μg of arachidonic acid, 10 μg of calcium ionophore A-23187 and the test compound (final concentrations 10 μM, 1 μM, 0.1 μM and 0.01 μM)], and the reaction was allowed to proceed at 37°C for 20 minutes. To the reaction mixture was added 4 mℓ of ethanol, which was shaken sufficiently, followed by leaving the resultant mixture standing for 10 minutes at room temperatures. The resultant mixture was subjected to centrifuge (2000 rpm) for 10 minutes, then the supernatant was separated. Thus-separated supernatant was concentrated to dryness under reduced pressure. To the concentrate was added 0.5 mℓ of a 60% aqueous methanol. 100μℓ Portion of this solution was taken and subjected to high performance liquid chromatography to perform quantitative determination of 5-HETE (5-hydroxyeicosatetraenoic acid). UV absorption of 5-HETE at 237 nm was measured with a UV absorption monitor. The inhibitory effect (IE) of 5-HETE is expressed by $(1\text{-}b/a) \times 100$ . In this formula, a means the height of the peak or the area of the peak in the case of containing no compound (I), while b means the height of the peak or the area of the peak in the case of containing the compound (I). The results revealed, as shown in Table 1, that the test compounds showed strong inhibitory action on the production of 5-HETE.

Table 1

| Effect of Inhibiting 5-Lipoxygenase | | | | |
|---|---|---|---|---|
| Compound | % Inhibition (IE) | | | |
| | $10^{-5}$M | $10^{-6}$M | $10^{-7}$M | $10^{-8}$M |
| 5 | 100 | 100 | 57 | 0 |
| 6 | 100 | 100 | 52 | 4 |
| 10 | 100 | 99 | 98 | 12 |
| 12 | 100 | 100 | 90 | 46 |

Experimental Example 2

Through the descending aorta of a Wistar rat, 10 mℓ of blood was collected, under anesthesia, together with citric acid of a volume corresponding to 10%. PRP and PPP were respectively prepared, then they were mixed to make the number of platelets to be $10^9$/mℓ. To 2.5 μℓ each of the test solutions prepared in advance (final concentrations of the test compounds : 100 μM, 10 μM, 1 μM and 0.1 μM) was added 0.225 mℓ of the platelet solution. The respective solutions were kept at 37°C for 5 minutes, to which was added 25 μℓ each of arachidonic acid solution (50 μg/mℓ) , then the respective mixtures were shaken immediately. The reaction was allowed to proceed at 37°C for 15 minutes, and there was added ethanol (1 mℓ) to stop the reaction. The reaction mixture was subjected to a centrifuge (2000 rpm) for 5 minutes. One mℓ of the supernatant was taken, which was mixed with 1 mℓ of water. 100μℓ of this mixture solution was subjected to high performance liquid chromatography to quantitatively determine 12-HETE. The detection was conducted at 240 nm. The calculation of the inhibition rate was conducted in a manner as in the case of 5-HETE. The results were shown in Table 2.

Table 2

| 12-Lipoxygenase Inhibitory Action | | | | |
|---|---|---|---|---|
| Compound | % Inhibition (IE) | | | |
| | $10^{-4}$M | $10^{-5}$M | $10^{-6}$M | $10^{-7}$M |
| 5 | 97 | 78 | 14 | - |
| 6 | 98 | 88 | 16 | 2 |
| 10 | 96 | 92 | 52 | 45 |
| 12 | 95 | 82 | 11 | - |
| 15 | 100 | 100 | 99 | 25 |
| 17 | 100 | 99 | 98 | 22 |
| 18 | 100 | 100 | 89 | 18 |
| 20 | 100 | 99 | 97 | 9 |
| 21 | 100 | 100 | 98 | 21 |
| 23 | 100 | 100 | 92 | 19 |
| 24 | 100 | 99 | 98 | 13 |
| 26 | 100 | 99 | 28 | - |
| 27 | 100 | 100 | 99 | 19 |
| 29 | 100 | 99 | 58 | 7 |
| 30 | 100 | 100 | 98 | 21 |
| 32 | 99 | 99 | 45 | 2 |
| 33 | 100 | 100 | 28 | 2 |
| 35 | 99 | 99 | 49 | 8 |
| 36 | 100 | 100 | 100 | 97 |
| 37 | 100 | 100 | 31 | 2 |
| 38 | 99 | 99 | 90 | 3 |
| 39 | 100 | 100 | 32 | - |
| 40 | 99 | 96 | 48 | 6 |
| 41 | 99 | 74 | 20 | 9 |

## Examples

By the following Reference Examples. Examples and Formulation Examples of the compounds of the present invention, the present invention will be described in a more concrete manner.

### Reference Example 1

To a solution of 4-acetoxy-2,3,5-trimethylphenol [20 g (103 mmol.)] and methalyl chloride [10 g (110.4 mmol.)] in dimethylformamide (160 mℓ) was added potassium carbonate [15.2 g (110 mmol.)]. The mixture was stirred for 3 hours at 80°C under argon atmosphere. The reaction mixture was, after cooling, diluted with water, which was subjected to extraction with ethyl acetate. The extract was washed with water and dried, then the solvent was distilled off. The residue was crystallized from hexane to obtain the desired 4-acetoxy-2,3,5-trimethylphenyl-2-methylpropenylether [18.5 g (yield 72.4%)], m.p.44° to 45°C.

In a similar manner to the above, 4-acetoxy-2,3,5-trimethylphenyl allyl ether was synthesized. (yield 76.7%, m.p. 40°- 41°C).

Reference Example 2

In N,N-diethylaniline (100 mℓ) was dissolved 4-acetoxy-2,3,5-trimethylphenyl 2-methylpropenylether [16.2 g (6.5 mmol)], which was heated at 200°C for two hours. The reaction mixture was cooled and diluted with isopropyl ether, which was washed with 2N-HCℓ to remove N,N-diethylaniline. The remainder was washed with a saturated aqueous solution of sodium hydrogencarbonate, which was dried, followed by distilling off the solvent. The residue was crystallized from isopropylether-hexane to obtain the desired 4-acetoxy-2-(2-methyl-2-propenyl)-3,5,6-trimethylphenol [14.9 g (yield 91.7%)] , m.p. 109°-110°C.

In a manner similar to the above, 4-acetoxy-2-allyl-3,4,6-trimethylphenol was synthesized. (Yield 94.6%, m.p. 117°-118°C)

Reference Example 3

In methylene chloride (100 mℓ) was dissolved 4-acetoxy-2-(2-methyl-2-propenyl)-3,5,6-trimethylphenol [30 g (40.3 mmol)]. To the solution was added at 0 C, in limited amounts, m-chloroperbenzoic acid [16.7 g (67.8 mmol)]. The reaction mixture was stirred for one hour, to which was added triethylamine (30 mℓ), followed by stirring for further one hour. The reaction mixture was washed with a saturated aqueous solution of sodium hydrogen carbonate, which was dried, followed by distilling off the solvent. The residue was crystallized from isopropyl ether - hexane to obtain the desired 5-acetoxy-2-hydroxymethyl-2,4,6,7-tetramethyl-2,3-dihydrobenzofuran [9.3 g (yield 87.4%, m.p. 98-99°C)].

Reference Example 4

To a methylene chloride solution (50 mℓ) of oxalyl chloride (2 mℓ) was added dropwise at -60°C dimethylsulfoxide (4 mℓ). The mixture was stirred for 10 minutes, to which was then added dropwise a methylene chloride (10 mℓ) of 5-acetoxy-2-hydroxymethyl-2,4,6,7-tetramethyl-2,3-dihyrobenzofuran [5.0 g (19 mmol)]. The reaction mixture was stirred for 15 minutes, to which was added triethylamine (15 mℓ), and the mixture was stirred for further 10 minutes. The reaction mixture was washed with water and dried, followed by distilling off the solvent. The residue was crystallized from isopropylether - hexane to obtain 5-acetoxy-2-formyl-2,4,6,7-tetramethyl-2,3-dihydrobenzofuran (yield 96.7%), m.p. 78-79°C.

Example 1

To a chloroform (15 mℓ) solution of 4-acetoxy-2-allyl-3,5,6-trimethylphenol [2.0 g(8.5 mmol)] was added dropwise, while stirring, bromine [1.36 g (8.5 mmol)]. To the mixture was then added triethylamine (0.3 mℓ), which was heated for two hours under reflux. The reaction mixture was cooled, washed with water, dried and then concentrated. The concentrate was crystallized from hexane to obtain 5-acetoxy-2-bromomethyl-4,6,7-trimethyl-2,3-dihydrobenzofuran (Compound 14) [2.5 g(yield 93.2%)].

In a manner similar to the above, 5-acetoxy-2-bromomethyl-2,4,6,7-tetramethyl-2,3-dihydrobenzofuran (Compound 13) was obtained from 4-acetoxy-3,5,6-trimethyl-2-(2-methyl-2-propenyl)phenol.

Example 2

To a dimethyl sulfoxide (5 mℓ) solution of 5-acetoxy-2-bromomethyl-2,4,6,7-tetramethyl-2,3-dihydrobenzofuran [1.5 g(4.58 mmol)] was added sodium cyanide [270 mg (5.5 mmol)], and the mixture was stirred at 80°C for 6 hours under argon atmosphere. The reaction mixture was, after cooling, diluted with water, which was subjected to extraction with ethyl acetate. The extract was washed with water and dried, followed by distilling off the solvent. The residue was purified by means of a silica gel column chromatography [hexane - isopropyl ether (2:1)] to obtain 2-bromomethyl-5-hydroxy-2,4,6,7-tetramethyl-2,3-dhydrobenzofuran (Compound 6) (350 mg) and 2-cyanomethyl-5-hydroxy-2,4,6,7-tetramethyl-2,3-dihydrobenzofuran (Compound 5)(158 mg).

Example 3

To a dimethylformamide (20 ml) solution of triethyl phosphonoacetate [2.7 g (12.1 mmol)] was added sodium hydride (content 60%)(504 mg), which was stirred for 15 minutes. To the reaction mixture was added a dimethyl formamide (5 mℓ) solution of 5-acetoxy-2-formyl-2,4,6,7-tetramethyl-2,3-dihydrobenzofuran [3.0 g (11.5 mmol)], and the mixture was stirred for further 30 minutes. The reaction mixture was diluted with water, which was subjected to extraction with ethyl acetate. The extract solution was washed with water and dried, followed by distilling off the solvent. The residue was crystallized from hexane-isopropylether to obtain 3-(5-acetoxy-2,4,6,7-pentamethyl-2,3-dihydrobenzofuran-2-yl)acetic acid ethyl ester (Compound 4)(3.5 g). In a manner similar to the above, Compound 8 was synthesized.

Example 4

To a methanol (10 mℓ) solution of ethyl ester of 3-(5-acetoxy-2,4,6,7-tetramethyl-2,3-dihydrobenzofuran-2-yl) acrylic acid [3.5 g (10.5 mmol)] was added a solution of sodium hydroxide (1.0 g) in water (5 mℓ). The mixture was heated for two hours under reflux under argon atmosphere. The reaction mixture was cooled and neutralized with 2N-HCℓ, which was subjected to extraction with isopropyl ether. The extract solution was washed with water and dried, followed by distilling off the solvent. The residue was crystallized from tetrahydrofuran - ethyl acetate to afford the desired 3-(5-acetoxy-2,4,6,7-pentamethyl-2,3-dihydrobenzo furan-2-yl)acrylic acid (Compound 3)(2.06 g). In a manner similar to the above, Compounds 7 and 10 were synthesized.

Example 5

To a solution of 3-(5-acetoxy-2,4,6,7-tetramethyl-2,3-dihydrobenzofuran-2-yl)acrylic acid (1.0 g) in acetic acid (10 mℓ) was added 5% palladium-carbon (0.4 g), and the mixture was subjected to hydrogenation for two hours at 80 C. The catalyst was then filtered off, followed by distilling off the solvent. The residue was crystallized from tetrahydrofuran-isopropyl ether (IPE) to afford the desired 3-(5-hydroxy-2,4,6,7-pentamethyl-2,3-dihydrobenzofuran-2-yl) propionic acid (Compound 2)(0.97 g). In a manner similar to the above, Compounds 9 and 12 were obtained.

Example 6

To a solution of 5-hydroxy-2-cyanomethyl-2,4,6,7-tetramethyl-2,3-dihydrobenzofuran in methanol (5 mℓ) was added a solution of sodium hydroxide (0.5 g) in water (5 mℓ). The mixture was heated for three hours under reflux under argon atmosphere. The reaction mixture was cooled and neutralized with 2N-HCℓ. The reaction product was extracted with ethyl acetate. The extract solution was washed with water, dried and concentrated, which was crystallized from ethyl acetate - isopropyl ether to afford the desired 2-(5-hydroxy-2,4,6,7-tetramethyl-2,3-dihydrobenzofuran-2-yl)acetic acid (Compound 1) (0.1 g).

Example 7

To a suspension of benzyltriphenyl phosphonium chloride [1.5 g (3.87 mmol)] in tetrahydrofuran (5 mℓ) was added dropwise, under ice-cooling, an n-butyl lithium hexane solution [(1.6 M) 24 mℓ], followed by stirring for 15 minutes. To the reaction mixture was added a solution of 5-acetoxy-2-formyl-2,4,6,7-tetramethyl-2,3-dihydrobenzofuran (1.0 g) in tetrahydrofuran (3 mℓ). The mixture was added water, which was subjected to extraction with ethyl acetate. The extract solution was washed with water and dried, then the solvent was distilled off. The residue was purified by means of a silica gel column chromatography [hexane-IPE(2:1)] to obtain the desired 5-acetoxy-2-cinnamoyl-2,4,6,7-tetramethyl-2,3-dihydrobenzofuran (Compound 11)(1.22 g).

Physico-chemical properties of the compounds obtained as above are shown in Table 3.

Table 3

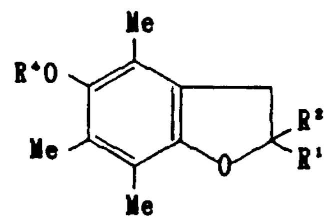

| Compd. No. | $R^1$ | $R^2$ | $R^4$ | Yield (%) | m.p. (°C) | NMR (δ ppm) $CDC\ell_3$中 |
|---|---|---|---|---|---|---|
| 1 | Me | $-CH_2CO_2H$ | H | 22.7 | 184-185 | 1.47(3H), 1.98(3H), 2.03(6H), 2.63(2H), 2.80(1H), 3.20(1H), 4.00(1H), 7.10(1H); in DMSO-$d_6$ |
| 2 | Me | $-(CH_2)_2CO_2H$ | H | 96.3 | 164-165 | 1.33(3H), 1.85(2H), 1.97(3H), 2.03(6H), 2.27(2H), 2.73(1H), 2.95(1H), 3.50(1H), 7.20(1H); in DMSO-$d_6$ |
| 3 | Me | $-CH=CHCO_2H$ (E) | H | 74.6 | 211-212 | 1.53(3H), 2.05(6H), 3.03(2H), 3.60(1H), 5.83(1H), 6.92(1H), 7.10(1H); in DMSO-$d_6$ |
| 4 | Me | $-CH=CHCO_2Et$ (E) | Ac | 92.0 | 91- 92 | 1.27(3H), 1.58(3H), 1.95(3H), 2.00(3H), 2.13(3H), 2.30(3H), 3.07(2H), 4.17(2H), 6.02(1H), 7.05(1H) |

| | | | | | | |
|---|---|---|---|---|---|---|
| 5 | Me | $-CH_2CN$ | H | 11.7 | 127-128 | 1.63(3H), 2.10(9H), 2.68(2H), 2.95(1H), 3.17(1H), 4.20(1H) |
| 6 | Me | $-CH_2Br$ | H | 26.8 | 101-102 | 1.60(3H), 2.12(9H), 2.88(1H), 3.27(1H), 3.48(2H), 4.15(1H) |
| 7 | Me | $-(CH=CH)_2-CO_2H$ E | H | 82.9 | 204-206 | 1.50(3H), 2.03(9H), 3.00(2H), 3.90(1H), 5.87(1H), 6.37(2H), 7.15(1H); in DMSO-$d_6$ |
| 8 | Me | $-(CH=CH)_2-CO_2Et$ E | Ac | 87.8 | — | 1.27(3H), 1.57(3H), 1.97(3H), 2.03(3H), 2.13(3H), 2.33(3H), 3.05(2H), 4.17(2H), 5.87(1H), 6.37(2H), 7.20(1H) |
| 9 | Me | $-(CH_2)_4CO_2H$ | H | 74.0 | 143-144 | 1.28(3H), 1.50(6H), 1.93(3H), 2.00(6H), 2.20(2H), 2.72(1H), 2.93(1H), 7.20(1H) ;in DMSO-$d_6$ |
| 10 | Me | $-CH=CH-Ph$ E, Z | H | 95.1 | — | 1.52(3H), 1.87(3H), 2.00(3H), 2.07(3H), 2.88(1H), 3.18(1H), 4.08(1H), 5.90(1H), 6.48(1H), 7.23(5H) |

| | | | | | | |
|---|---|---|---|---|---|---|
| 11 | Me | -CH=CH-Ph (E, Z) | Ac | 95.2 | — | 1.57(3H), 1.90(6H), 1.97(3H), 2.30(3H), 2.88(1H), 3.20(1H), 5.90(1H), 6.50(1H), 7.25(5H) |
| 12 | Me | $-(CH_2)_2-Ph$ | H | 85.7 | 80- 81 | 1.47(3H), 1.98(2H), 2.13(6H), 2.15(3H), 2.73(2H), 2.87(1H), 3.05(1H), 4.08(1H), 7.23(5H) |
| 13 | Me | $-CH_2Br$ | Ac | 91.0 | — | 1.60(3H), 2.00(6H), 2.10(3H), 2.30(3H), 2.90(1H), 3.30(1H), 3.50(2H) |
| 14 | H | $-CH_2Br$ | Ac | 93.2 | 80- 81 | 2.00(6H), 2.10(3H), 2.30(3H), 3.13(2H), 3.53(2H), 4.97(1H) |
| 15 | Me | $-CH=CH-CH_2-Ph$ (Z) | H | 70.4 | 62- 63 | 1.57(3H), 2.11(3H), 2.16(3H), 2.18(3H), 3.06(1H), 3.20(1H), 3.56(1H), 3.69(1H), 4.16(1H), 5.56(1H), 5.75(1H), 7.15-7.35 (5H) |

| | | | | | | |
|---|---|---|---|---|---|---|
| 16 | Me | $-CH\underset{Z}{=}CH-CH_2-Ph$ | Ac | 74.9 | oil | 1.60(3H), 2.00(6H), 2.08(3H), 2.32(3H), 3.05(1H), 3.23(1H), 3.56(1H), 3.67(1H), 5.50(1H), 5.75(1H), 7.23(5H, m) |
| 17 | Me | $-CH\underset{E}{=}CH-CH_2-Ph$ | H | 50.0 | 97- 98 | 1.52(3H), 2.10(3H), 2.12(3H), 2.14(3H), 2.95(1H), 3.10(1H), 3.38(2H), 4.15(1H), 5.74(1H), 5.84(1H), 7.10-7.40(5H, m) |
| 18 | Me | $-CH\underset{E}{=}CH-Ph$ | H | 61.0 | 107-108 | 1.64(3H), 2.11(3H), 2.16(3H), 2.18(3H), 3.06(1H), 3.20(1H), 4.17(1H), 6.40(1H), 6.64(1H), 7.15-7.45(5H) |
| 19 | Me | $-CH\underset{E}{=}CH-Ph$ | Ac | 92.0 | 103-105 | 1.63(3H), 1.97(3H), 2.02(3H), 2.15(3H), 2.30(3H), 3.00(1H), 3.23(1H), 6.33(1H), 6.65(1H), 7.20-7.50(5H) |
| 20 | Me | $-(CH_2)_3-Ph$ | H | 93.3 | 92- 93 | 1.37(3H), 1.72(4H), 2.07(6H), 2.10(3H), 2.62(2H), 2.77(1H), 2.97(1H), 4.07(1H), 7.00-7.35(5H) |

| | | | | | | |
|---|---|---|---|---|---|---|
| 21 | Me | -CH=CH-$(CH_2)_2$-Ph (Z) | H | 95.5 | 64- 65 | 1.45(3H), 2.07(6H), 2.12(3H), 2.61(4H), 3.00(2H), 4.08(1H), 5.48(1H), 6.14(1H), 7.00-7.30(5H) |
| 22 | Me | -CH=CH-$(CH_2)_2$-Ph (Z) | Ac | 57.6 | oil | 1.47(3H), 1.93(3H), 1.98(3H), 2.07(3H), 2.30(3H), 2.40-2.80(2H), 3.00(2H), 5.38(1H), 5.68(1H), 7.00-7.35(5H) |
| 23 | Me | -$(CH_2)_4$-Ph | H | 87.5 | 73- 74 | 1.20-1.80(6H), 1.03(3H), 1.07(6H), 2.12(3H), 2.60(2H), 2.77(1H), 2.97(1H), 4.08(1H), 7.00-7.35(5H) |
| 24 | Me | -CH=CH-Ph-4-OMe (Z) | H | 97.0 | oil | 1.53(3H), 1.93(3H), 2.02(3H), 2.08(3H), 2.90(1H), 3.22(1H), 3.78(3H), 4.17(1H), 5.83(1H), 6.42(1H), 6.78(2H), 7.23(2H) |
| 25 | Me | -CH=CH-Ph-4-OMe (Z) | Ac | 81.1 | oil | 1.53(3H), 1.92(6H), 1.97(3H), 2.27(3H), 2.90(1H), 3.22(1H), 3.77(3H), 5.83(1H), 6.42(1H), 6.77(2H), 7.20(2H) |

| | | | | | | |
|---|---|---|---|---|---|---|
| 26 | Me | $-(CH_2)_2$-Ph-4-OMe | H | 93.8 | 77- 78 | 1.47(3H), 1.65-2.10(2H), 2.13(6H), 2.15(3H), 2.40-2.80(2H), 2.83(1H), 3.05(1H), 3.78(3H), 4.10(1H), 6.78(2H), 7.10(2H) |
| 27 | Me | -CH=CH-Ph-3-OMe (Z) | H | 98.0 | oil | 1.53(3H), 1.90(3H), 2.02(3H), 2.08(3H), 2.90(1H), 3.22(1H), 3.67(3H), 4.17(1H), 5.90(1H), 6.48(1H), 6.65-7.30(4H) |
| 28 | Me | -CH=CH-Ph-3-OMe (Z) | Ac | 92.6 | oil | 1.53(3H), 1.88(6H), 1.95(3H), 2.90(1H), 3.23(1H), 3.67(3H), 5.88(1H), 6.47(1H), 6.60-6.90(3H), 7.05-7.30(1H) |
| 29 | Me | $-(CH_2)_2$-Ph-3-OMe | H | 84.2 | 79- 80 | 1.47(3H), 1.70-2.10(2H), 2.10(6H), 2.13(3H), 2.40-2.80(2H), 2.83(1H), 3.05(1H), 3.77(3H), 4.10(1H), 6.60-6.85(3H) 7.10-7.30(1H) |

| | | | | | | |
|---|---|---|---|---|---|---|
| 30 | Me | -CH=CH-Ph-4-F<br>Z | H | 94.6 | oil | 1.53(3H), 1.85(3H), 2.03(3H), 2.07(3H), 2.90(1H), 3.20(1H), 4.20(1H), 5.85(1H), 6.43(1H), 6.75-7.10(2H), 7.10-7.30(2H) |
| 31 | Me | -CH=CH-Ph-4-F<br>Z | Ac | 88.8 | oil | 1.55(3H), 1.83(3H), 1.90(3H), 1.93(3H), 2.27(3H), 2.90(1H), 3.22(1H), 5.87(1H), 6.42(1H), 6.90(2H), 7.20(2H) |
| 32 | Me | $-(CH_2)_2$-Ph-4-F | H | 79.6 | 76- 77 | 1.47(3H), 1.70-2.10(2H), 2.10(6H), 2.13 (3H), 2.40-2.80(2H), 2.83(1H), 3.05(1H) 4.10(1H), 7.10(4H) |
| 33 | Me | -CH=CH-Ph-4-Me<br>Z | H | 96.5 | oil | 1.53(3H), 1.9Q(6H), 1.97(3H), 2.28(3H), 2.33(3H), 2.90(1H), 3.22(1H), 5.87(1H), 6.47(1H), 6.95-7.25(4H) |
| 34 | Me | -CH=CH-Ph-4-Me<br>Z | Ac | 91.1 | oil | 1.53(3H), 1.90(3H), 2.00(3H), 2.07(3H), 2.88(1H), 3.20(1H), 4.10(1H), 5.87(1H), 6.43(1H), 6.90-7.30(4H) |

| | | | | | | |
|---|---|---|---|---|---|---|
| 35 | Me | $-(CH_2)_2-Ph-4-Me$ | H | 80.5 | 106-107 | 1.47(3H), 1.70-2.10(2H), 2.10(6H), 2.13(3H), 2.40-2.80(2H), 2.83(1H), 3.05(1H), 4.10(1H), 7.10(4H) |
| 36 | Me | $-CH\underset{Z}{=}CH-(CH_2)_2Me$ | H | 41.5 | oil | 0.91(3H), 1.40(2H), 1.52(3H), 2.12(6H), 2.17(3H), 2.19(2H), 3.04(2H), 3.16(1H), 4.16(1H), 5.38(1H), 5.68(1H) |
| 37 | Me | $-(CH_2)_4Me$ | H | 90.4 | 59- 60 | 0.88(3H), 1.29(6H), 1.39(3H), 1.68(6H), 2.10(6H), 2.13(3H), 2.81(1H), 2.97(1H), 4.14(1H) |
| 38 | Me | $-CH\underset{Z}{=}CH-(CH_2)_6Me$ | H | 49.7 | oil | 0.88(3H), 1.26(10H), 1.51(3H), 2.12(6H), 2.14(3H), 2.20(2H), 3.04(2H), 3.16(1H), 4.15(1H), 5.38(1H), 5.66(1H) |
| 39 | Me | $-(CH_2)_8Me$ | H | 83.3 | 69- 70 | 0.88(3H), 1.26(14H), 1.39(3H), 1.70 (2H), 2.10(6H), 2.13(3H), 2.81(1H), 2.97(1H), 4.13(1H) |

| | | | | | | |
|---|---|---|---|---|---|---|
| 40 | Me | $-CH{=}CH-(CH_2)_{10}Me$ (Z) | H | 45.8 | oil | 0.88(3H), 1.25(18H), 1.51(3H), 2.11(6H), 2.14(3H), 2.20(2H), 3.04(2H), 3.16(1H), 4.14(1H), 5.38(1H), 5.66(1H) |
| 41 | Me | $-(CH_2)_{12}Me$ | H | 84.6 | 67- 68 | 0.88(3H), 1.25(22H), 1.39(3H), 1.68(2H), 2.10(6H), 2.13(3H), 2.81(1H), 2.97(1H), 4.14(1H) |

Me: Methyl, Ac: Acetyl, Ph: Phenyl

Formulation Examples

A) Soft capsule

| | |
|---|---|
| (1) Compound 3 | 50 mg |
| (2) Corn oil | 100 mg |
| | total 150 mg |

By a conventional method, (1) and (2) were mixed, which was filled in a capsule.

B) Tablet

| | |
|---|---|
| (1) Compound 10 | 50 mg |
| (2) Lactose | 30 mg |
| (3) Corn starch | 10.6 mg |
| (4) Corn starch (paste) | 5 mg |
| (5) Magnesium stearate | 0.4 mg |
| (6) Carboxymethyl cellulose sodium | 20 mg |
| | total 116 mg |

By a conventional method, these were mixed, which was tableted by a tablet machine.

## Claims

**Claims for the following Contracting States : AT, BE, CH, DE, FR, GB, IT, LI, LU, NL, SE**

**1.** A compound of the formula:

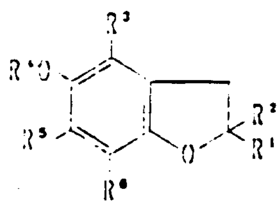

(I)

wherein $R^1$ is hydrogen or $C_{1-6}$ alkyl; $R^2$ is methyl which is substituted by carboxy, $C_{2-5}$ alkoxycarbonyl, cyano, halogen, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with one or more substituents selected from $C_{1-20}$ alkyl. $C_{1-6}$ alkyl substituted by hydroxyl, carboxyl, $c_{2-5}$ alkoxycarbonyl, piperazyl or phenylthio, $c_{2-4}$ alkenyl, halogen, nitro, formyl, $C_{1-3}$ alkoxy carboxy, trifluoromethyl, di-$C_{1-3}$-alkylamino, $C_{5-7}$ cycloalkyl and $C_{1-3}$ alkylthio; or $R^2$ is a $C_{2-15}$ alkyl or $C_{2-15}$ alkenyl group which may be substituted by carboxy, $c_{2-5}$ alkoxycarbonyl, halogen, cyano, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, $c_{1-10}$ alkanoyl or cyclic $C_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $c_{1-3}$ alkoxy, hydroxyl, nitro and halogen,

or a salt thereof, with the exclusion of 5-hydroxy-4,6,7-trimethyl-2-isopropyl-2,3-dihydrobenzofuran, 5-acetoxy-4,6,7-trimethyl-2-isopropyl-2,3-dihydrobenzofuran, 5-hydroxy-4,6,7-trimethyl-2-n-propyl-2,3-dihydrobenzofuran, 5-hydroxy-4,6,7-trimethyl-2-ethyl-2,3-dihydrobenzofuran, 5-hydroxy-4,6,7-trimethyl-2-n-tridecyl-2,3-dihydrobenzofuran, and 5-hydroxy-2,4,6,7-tetramethyl-2-(3',7',11'-trimethyl-dodecyl)-2,3-dihydrobenzofuran.

**2.** A compound of the formula:

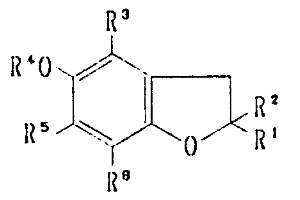

(I)

wherein $R^1$ is hydrogen or $C_{1-6}$ alkyl; $R^2$ is methyl which is substituted by carboxy, $C_{2-5}$ alkoxycarbonyl, cyano, halogen, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with one or more substituents selected from $C_{1-20}$ alkyl, $C_{1-6}$ alkyl substituted by hydroxyl, carboxyl, $c_{2-5}$ alkoxycarbonyl, piperazyl or phenylthio, $c_{2-4}$ alkenyl, halogen, nitro, formyl, $C_{1-3}$ alkoxy, carboxy, trifluoromethyl, di-$C_{1-3}$-alkylamino, $C_{5-7}$ cycloalkyl and $C_{1-3}$ alkylthio; or $R^2$ is a $C_{2-15}$ alkyl or $C_{2-15}$ alkenyl group which may be substituted by carboxy, $c_{2-5}$ alkoxycarbonyl, halogen, cyano, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, $c_{1-10}$ alkanoyl or cyclic $C_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $c_{1-3}$ alkoxy, hydroxyl, nitro and halogen, or a salt thereof, with the exclusion of 5-hydroxy-2,4,6,7-tetramethyl-2-(3',7',11'-trimethyl-dodecyl)-2,3-dihydrobenzofuran, as a medicament for the treatment of the human or animal body by therapy.

**3.** A compound according to claim 2 whereby the medicament is for ameliorating the circulatory system, for treating the central nervous system, or for treating allergic diseases.

**4.** A compound of claim 3 as a 5-lipoxygenase and/or 12-lipoxygenase inhibitor.

**5.** A compound according to claim 1, 2 or 3 wherein $R^1$ is hydrogen or $C_{1-3}$ alkyl.

**6.** A compound according to claim 1, 2 or 3 wherein $R^2$ is methyl substituted by carboxy, cyano or halogen.

**7.** A compound according to claim 1, 2 or 3 wherein $R^2$ is methyl substituted by phenyl, thienyl, pyridyl, imidazolyl, thiazolyl or morpholino which may be further substituted by $C_{1-20}$ alkyl, $C_{1-3}$ alkyl substituted by hydroxy, carboxyl, $C_{2-5}$ alkoxycarbonyl, piperazyl or phenylthio, $C_{2-4}$ alkenyl, hydroxy, halogen, nitro, formyl, $C_{1-3}$ alkoxy, carboxy, trifluoromethyl, di-$C_{1-3}$ alkylamino, $C_{5-7}$ cycloalkyl or $C_{1-3}$ alkylthio.

**8.** A compound according to claim 1, 2 or 3 wherein $R^2$ is $C_{2-15}$ alkyl or $C_{2-15}$ alkenyl which may be substituted by carboxy, $C_{2-5}$ alkoxycarbonyl or phenyl which may be substituted with halogen, $C_{1-3}$ alkyl, $C_{1-3}$ alkoxy or hydroxy.

**9.** A compound according to claim 1, 2 or 3 wherein $R^4$ is hydrogen or $C_{1-10}$ alkanoyl.

**10.** A compound according to claim 1, 2 or 3 wherein $R^5$ and $R^6$ are each $C_{1-6}$ alkyl or $C_{1-3}$ alkoxy.

**11.** A compound according to claim 1, 2 or 3 wherein $R^1$ is methyl; $R^2$ is $C_{2-6}$ alkyl or $C_{2-6}$ alkenyl which is substituted by phenyl which may be substituted by $C_{1-3}$ alkyl, $C_{1-3}$ alkoxy or halogen; $R^3$ is methyl; $R^4$ is hydrogen; and $R^5$ and $R^6$ are each methyl.

**12.** A compound according is claim 1, 2 or 3 which is 5-hydroxy-2-cinnamoyl-2,4,6,7-tetramethyl-2,3-dihydrobenzo-furan.

13. A compound according to claim 1, 2 or 3 which is 5-hydroxy-2-phenylethyl-2,4,6,7-tetramethyl-2,3-dihydrobenzofuran.

14. A compound according to claim 1, 2 or 3 which is 5-hydroxy-2-(1-(Z)-pentenyl)-2,4,6,7-tetramethyl-2,3-dihydrobenzofuran.

15. Use of a compound according to claim 1, 2 or 3 or a salt thereof for the preparation of a 5-lipoxygenase and/or 12-lipoxygenase inhibitor.

16. Use of a compound of the formula:

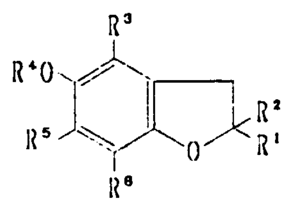

(I)

wherein $R^1$ is hydrogen or $C_{1-6}$ alkyl; $R^2$ is methyl which is substituted by carboxy, $C_{2-5}$ alkoxycarbonyl, cyano, halogen, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with one or more substituents selected from $C_{1-20}$ alkyl, $C_{1-6}$ alkyl substituted by hydroxyl, carboxy, $c_{2-5}$ alkoxycarbonyl, piperazyl or phenylthio, $c_{2-4}$ alkenyl, halogen, nitro, formyl, $C_{1-3}$ alkoxy, carboxy, trifluoromethyl, di-$C_{1-3}$-alkylamino, $C_{5-7}$ cycloalkyl and $C_{1-3}$ alkylthio; or $R^2$ is a $C_{2-15}$ alkyl or $C_{2-15}$ alkenyl group which may be substituted by carboxy, $c_{2-5}$ alkoxycarbonyl, halogen, cyano, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, $c_{1-10}$ alkanoyl or cyclic $C_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $c_{1-3}$ alkoxy, hydroxyl, nitro and halogen, or a salt thereof, for producing a medicament for ameliorating the circulatory system, for treating allergic diseases or for treating the central nervous system.

17. Use according to claim 16, wherein the medicament is a 5-lipoxygenase and/or 12-lipoxygenase inhibitor.

18. A method of producing a compound according to claim 1, which comprises reacting a compound of the formula:

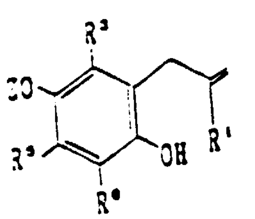

(II)

wherein $R^1$, $R^3$, $R^5$ and $R^6$ are of the same meaning defined as above, Z is hydrogen or a hydroxyl-protecting group, with a halogen molecule in the presence of a base to cause ring-closure, or by subjecting the compound (II) to the treatment with a peracid in the presence of a base to cause ring closure, followed by allowing thus ring-closed compound, in case of the 2-hydroxymethyl derivative, to react with an oxidizing agent, then subjecting thus-obtained compound to addition-elimination reaction with a compound represented by the formula

$$(C_6H_5)_3P^{\oplus}\text{-}R^2X^{\ominus} \quad \text{(III)}$$

wherein X is halogen, $R^2$ is a hydrocarbon residue whose carbon number is less by one than that of $R^2$, followed by, when desired, subjecting the resulting product to deprotection, acylation, hydrogenation or(and) substituent-exchange reaction, respectively.

**Claims for the following Contracting States : ES, GR**

1. A method of producing a compound of the formula:

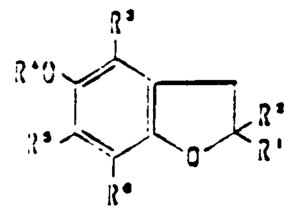

(I)

wherein $R^1$ is hydrogen or $C_{1-6}$ alkyl; $R^2$ is methyl which is substituted by carboxy, $C_{2-5}$ alkoxycarbonyl, cyano, halogen, phenyl, 1-naphthyl 2-naphthyl, indanyl, tetralyl, or a heterocyclic group selected from thienyl, pyridyl, imidazolyl, thialzolyl, morpholino and thiomorpholino, said phenyl or heterocyclic group optionally being further substituted with one or more substituents selected from $C_{1-20}$ alkyl, $C_{1-6}$ alkyl substituted by hydroxyl, carboxy, $C_{2-5}$ alkoxycarbonyl, piperazyl or phenylthio, $C_{2-4}$ alkenyl, halogen, nitro, formyl, $C_{1-3}$ alkoxy, carboxy, trifluoromethyl, di-$C_{1-3}$-alkylamino, $C_{5-7}$ cycloalkyl and $C_{1-3}$ alkylthio; or $R^2$ is a $C_{2-15}$ alkyl or $C_{2-15}$ alkenyl group which may be substituted by carboxy, $C_{2-5}$ alkoxycarbonyl, halogen, cyano, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, $C_{1-10}$ alkanoyl or cyclic $C_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $C_{1-3}$ alkoxy, hydroxyl, nitro and halogen, or a salt thereof, with the exclusion of 5-hydroxy-4,6,7-trimethyl-2-isopropyl-2,3-dihydrobenzofuran, 5-acetoxy-4,6,7-trimethyl-2-isopropyl-2,3-dihydrobenzofuran, 5-hydroxy-4,6,7-trimethyl-2-n-propyl-2,3-dihydrobenzofuran, 5-hydroxy-4,6,7-trimethyl-2-ethyl-2,3-dihydrobenzofuran, 5-hydroxy-4,6,7-trimethyl-2-n-tridecyl-2,3-dihydrobenzofuran, and 5-hydroxy-2,4,6,7-tetramethyl-2-(3',7',11'-trimethyl-dodecyl)-2,3-dihydrobenzofuran, which comprises reacting a compound of the formula:

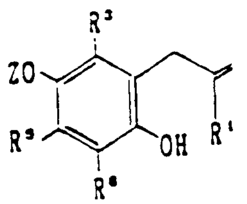

(II)

wherein $R^1$, $R^3$, $R^5$ and $R^6$ are of the same meaning defined as above, Z is hydrogen or a hydroxyl-protecting group, with a halogen molecule in the presence of a base to cause ring-closure, or by subjecting the compound (II) to the treatment with a peracid in the presence of a base to cause ring closure, followed by allowing thus ring-closed compound, in case of the 2-hydroxymethyl derivative, to react with an oxidizing agent, then subjecting thus-obtained compound to addition-elimination reaction with a compound represented by the formula

$$(C_6H_5)_3P^{\oplus}\text{-}R^{2'}X^{\ominus} \quad \text{(III)}$$

wherein X is halogen, $R^2$ is a hydrocarbon residue whose carbon number is less by one than that of $R^2$, followed by, when desired, subjecting the resulting product to deprotection, acylation, hydrogenation or-(and) substituent-exchange reaction, respectively.

2. A method for producing a medicament for ameliorating the circulatory system, for treating the central nervous system, or for treating allergic diseases which comprises mixing an effective 5-lipoxygenase and/or 12-lipoxygenase

inhibiting amount of a compound of the formula:

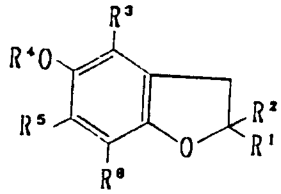

(I)

wherein $R^1$ is hydrogen or $C_{1-6}$ alkyl; $R^2$ is methyl which is substituted by carboxy, $C_{2-5}$ alkoxycarbonyl, cyano, halogen, phenyl, 1-naphthyl, 2-naphthyl, indanyl, tetralyl, or a heterocyclic group selected from thienyl, pyridyl, imidazolyl, thiazolyl, morpholino and thiomorpholino, said phenyl or heterocyclic group optionally being further substituted with one or more substituents selected from $C_{1-20}$ alkyl, $C_{1-6}$ alkyl substituted by hydroxyl, carboxyl, $c_{2-5}$ alkoxycarbonyl, piperazyl or phenylthio, $c_{2-4}$ alkenyl, halogen, nitro, formyl, $C_{1-3}$ alkoxy, carboxy, trifluoromethyl, di-$C_{1-3}$-alkylamino, $C_{5-7}$ cyaloalkyl and $C_{1-3}$ alkylthio; or $R^2$ is a $C_{2-15}$ alkyl or $C_{2-15}$ alkenyl group which may be substituted by carboxy, $c_{2-5}$ alkoxycarbonyl, halogen, cyano, phenyl, 1-naphthyl, 2-naphthyl, indahyl, tetralyl, or a heterocyclic group selected from thienyl, pyridyl, imidazolyl, thiazolyl, morpholino and thiomorpholino, said phenyl or heterocyclic group optionally being further substituted with halogen, $C_{1-3}$ alkyl, $C_{1-3}$ alkoxy or hydroxy, with the proviso that said alkyl or alkenyl group is unsubstituted at the carbon atom in position alpha to the benzofuran ring; $R^3$ is $C_{1-6}$ alkyl; $R^4$ is hydrogen, $c_{1-10}$ alkanoyl or cyclic $C_{3-10}$ alkanoyl; $R^5$ and $R^6$ are each $C_{1-6}$ alkyl or $C_{1-3}$ alkoxy, or $R^5$ and $R^6$, taken together, are butadienylene to form a condensed benzene ring which may be substituted by one to three substituents selected from the group consisting of a $C_{1-3}$ alkyl, $c_{1-3}$ alkoxy, hydroxyl, nitro and halogen, or a salt thereof, with the exclusion of 5-hydroxy-2,4,6,7-tetramethyl-2-(3',7',11'-trimethyl-dodecyl)-2,3-dihydrobenzofuran, with a per se known pharmaceutically acceptable carrier or excipient.

**3.** A method according to claim 2, wherein the medicament is a 5-lipoxygenase and/or 12-lipoxygenase inhibitor.

**4.** Method according to claim 1, 2 or 3, wherein $R^1$ is hydrogen or $C_{1-3}$ alkyl.

**5.** Method according to claim 1, 2 or 3, wherein $R^2$ is methyl substituted by carboxy, cyano or halogen.

**6.** Method according to claim 1, 2 or 3, wherein $R^2$ is methyl substituted by phenyl, thienyl, pyridyl, imidazolyl, thiazolyl or morpholino which may be further substituted by $C_{1-20}$ alkyl, $C_{1-3}$ alkyl substituted by hydroxy, carboxyl, $C_{2-5}$ alkoxycarbonyl, piperazyl or phenylthio, $C_{2-4}$ alkenyl, hydroxy, halogen, nitro, formyl, $C_{1-3}$ alkoxy, carboxy, trifluoromethyl, di-$C_{1-3}$ alkylamino, $C_{5-7}$ cycloalkyl or $c_{1-3}$ alkylthio.

**7.** Method according to claim 1, 2 or 3, wherein $R^2$ is $C_{2-15}$ alkyl or $C_{2-15}$ alkenyl which may be substituted by carboxy, $C_{2-5}$ alkoxycarbonyl or phenyl which may be substituted with halogen, $C_{1-3}$ alkyl, $C_{1-3}$ alkoxy or hydroxy.

**8.** Method according to claim 1, 2 or 3, wherein $R^4$ is hydrogen or $C_{1-10}$ alkanoyl.

**9.** Method according to claim 1, 2 or 3, wherein $R^5$ and $R^6$ are each $C_{1-6}$ alkyl or $C_{1-3}$ alkoxy.

**10.** Method according to claim 1, 2 or 3, wherein $R^1$ is methyl; $R^2$ is $C_{2-6}$ alkyl or $C_{2-6}$ alkenyl which is substituted by phenyl which may be substituted by $C_{1-3}$ alkyl, $C_{1-3}$ alkoxy or halogen; $R^3$ is methyl; $R^4$ is hydrogen; and $R^5$ and $R^6$ are each methyl.

**11.** Method according to claim 1, 2 or 3, wherein the compound is 5-hydroxy-2-cinnamoyl-2,4,6,7-tetramethyl-2,3-dihydrobenzo-furan.

**12.** Method according to claim 1, 2 or 3, wherein the compound is 5-hydroxy-2-phenylethyl-2,4,6,7-tetramethyl-2,3-dihydrobenzo-furan.

**13.** Method according to claim 1, 2 or 3, wherein the compound is 5-hydroxy-2-(1-(Z)-pentenyl)-2,4,6,7-tetramethyl-2,3-dihydrobenzofuran.

**14.** Use of a compound as defined in claim 2 or a salt thereof or of 5-hydroxy-2,4,6,7-tetramethyl-2-(3',7',11'-trimethyl-dodecyl)-2,3-dihydrobenzofuran as a component in the preparation of a medicament for inhibiting 5-lipoxygenase and/or 12-lipoxygenase.

## Patentansprüche

### Patentansprüche für folgende Vertragsstaaten : AT, BE, CH, DE, FR, GB, IT, LI, LU, NL, SE

1. Verbindung der Formel:

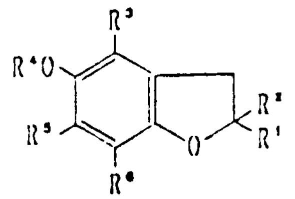

(I)

worin $R^1$ Wasserstoff oder $C_{1-6}$ Alkyl ist; $R^2$ Methyl ist, das durch Carboxy, $C_{2-5}$ Alkoxycarbonyl, Cyano, Halogen, Phenyl, 1-Naphthyl, 2-Naphtyl, Indanyl, Tetralyl oder eine heterocyclische Gruppe, ausgewählt aus Thienyl, Pyridyl, Imidazolyl, Thiazolyl, Morpholino und Thiomorpholino substituiert ist, wobei die Phenyl- oder heterocyclische Gruppe gegebenenfalls weiter substituiert ist mit einem oder mehreren Substituenten, ausgewählt aus $C_{1-20}$ Alkyl, $C_{1-6}$ Alkyl, substituiert durch Hydroxyl, Carboxyl, $C_{2-5}$ Alkoxycarbonyl, Piperazyl oder Phenylthio, $C_{2-4}$ Alkenyl, Halogen, Nitro, Formyl, $C_{1-3}$ Alkoxy, Carboxy, Trifluormethyl, Di-$C_{1-3}$ Alkylamino, $C_{5-7}$ Cycloalkyl und $C_{1-3}$ Alkylthio; oder $R^2$ eine $C_{2-15}$ Alkyl- oder $C_{2-15}$ Alkenylgruppe bedeutet, die substituiert sein kann durch Carboxy, $C_{2-5}$ Alkoxycarbonyl, Halogen, Cyano, Phenyl, 1-Naphthyl, 2-Naphthyl, Indanyl, Tetralyl oder eine heterocyclische Gruppe ausgewählt aus Thienyl, Pyridyl, Imidazolyl, Thiazolyl, Morpholino und Thiomorpholino, wobei die Phenyl- oder heterocyclische Gruppe gegebenenfalls weiter substituiert sein kann durch Halogen, $C_{1-3}$ Alkyl, $C_{1-3}$ Alkoxy oder Hydroxy, mit der Maßgabe, daß die Alkyl- oder Alkenylgruppe nicht substituiert ist an dem Kohlenstoffatom in der alpha-Position zum Benzofuranring; $R^3$ $C_{1-6}$ Alkyl ist; $R^4$ Wasserstoff, $C_{1-10}$ Alkanoyl oder cyclisches $C_{3-10}$ Alkanoyl ist; $R^5$ und $R^6$ jeweils $C_{1-6}$ Alkyl oder $C_{1-3}$ Alkoxy bedeuten, oder $R^5$ und $R^6$ zusammengenommen Butadienylen bedeuten, wobei ein kondensierter Benzolring gebildet wird, der substituiert sein kann durch 1 bis 3 Substituenten, ausgewählt aus der Gruppe, die besteht aus $C_{1-3}$ Alkyl, $C_{1-3}$ Alkoxy, Hydroxyl, Nitro und Halogen, oder ein Salz davon, mit Ausnahme von 5-Hydroxy-4,6,7-trimethyl-2-isopropyl-2,3-dihydrobenzofuran, 5-Acetoxy-4,6,7-trimethyl-2-isopropyl-2,3-dihydrobenzofuran, 5-Hydroxy-4,6,7-trimethyl-2-n-propyl-2,3-dihydrobenzofuran, 5-Hydroxy-4,6,7-trimethyl-2-ethyl-2,3-dihydrobenzofuran, 5-Hydroxy-4,6,7-trimethyl-2-n-tridecyl-2,3-dihydrobenzofuran, und 5-Hydroxy-2,4,6,7-tetramethyl-2-(3',7',11'-trimethyl-dodecyl)-2,3-dihydrobenzofuran.

2. Verbindung der Formel:

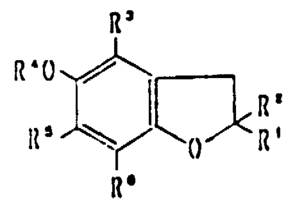

(I)

worin $R^1$ Wasserstoff oder $C_{1-6}$ Alkyl ist; $R^2$ Methyl ist, das durch Carboxy, $C_{2-5}$ Alkoxycarbonyl, Cyano, Halogen, Phenyl, 1-Naphthyl, 2-Naphtyl, Indanyl, Tetralyl oder eine heterocyclische Gruppe, ausgewählt aus Thienyl, Pyridyl, Imidazolyl, Thiazolyl, Morpholino und Thiomorpholino substituiert ist, wobei die Phenyl- oder heterocyclische Gruppe gegebenenfalls weiter substituiert ist mit einem oder mehreren Substituenten, ausgewählt aus $C_{1-20}$ Alkyl, $C_{1-6}$ Alkyl, substituiert durch Hydroxyl, Carboxyl, $C_{2-5}$ Alkoxycarbonyl, Piperazyl oder Phenylthio, $C_{2-4}$ Alkenyl, Halogen, Nitro, Formyl, $C_{1-3}$ Alkoxy, Carboxy, Trifluormethyl, Di-$C_{1-3}$ Alkylamino, $C_{5-7}$ Cycloalkyl und $C_{1-3}$ Alkylthio; oder $R^2$ eine $C_{2-15}$ Alkyl- oder $C_{2-15}$ Alkenylgruppe bedeutet, die substituiert sein kann durch Carboxy, $C_{2-5}$ Alkoxycarbonyl, Halogen, Cyano, Phenyl, 1-Naphthyl, 2-Naphthyl, Indanyl, Tetralyl oder eine heterocyclische Gruppe ausgewählt aus Thienyl, Pyridyl, Imidazolyl, Thiazolyl, Morpholino und Thiomorpholino, wobei die Phenyl- oder heterocyclische Gruppe gegebenenfalls weiter substituiert sein kann durch Halogen, $C_{1-3}$ Alkyl, $C_{1-3}$ Alkoxy oder Hydroxy, mit der Maßgabe, daß die Alkyl- oder Alkenylgruppe nicht substituiert ist an dem Kohlenstoffatom in der alpha-Position zum Benzofuranring; $R^3$ $C_{1-6}$ Alkyl ist; $R^4$ Wasserstoff, $C_{1-10}$ Alkanoyl oder cyclisches $C_{3-10}$ Alkanoyl ist; $R^5$ und $R^6$ jeweils $C_{1-6}$ Alkyl oder $C_{1-3}$ Alkoxy bedeuten, oder $R^5$ und $R^6$ zusammengenommen Butadienylen bedeuten, wobei ein kondensierter Benzolring gebildet wird, der substituiert sein kann durch 1 bis 3 Substituenten, ausgewählt aus der Gruppe, die besteht aus $C_{1-3}$ Alkyl, $C_{1-3}$ Alkoxy, Hydroxyl, Nitro und Halogen, oder ein Salz davon, mit Ausnahme von 5-Hydroxy-2,4,6,7-tetramethyl-2-(3',7',11'-trimethyl-dodecyl)-2,3-dihydrobenzofuran, als Medikament zur Behandlung des menschlichen oder tierischen Körpers.

3. Verbindung nach Anspruch 2, wobei das Medikament zur Verbesserung des Kreislaufsystems, zur Behandlung des zentralen Nervensystems oder zur Behandlung allergischer Krankheiten dient.

4. Verbindung nach Anspruch 3, als 5-Lipoxygenase- und/oder 12-Lipoxygenaseinhibitor.

5. Verbindung nach Anspruch 1, 2 oder 3, worin $R^1$ Wasserstoff oder $C_{1-3}$ Alkyl ist.

6. Verbindung nach Anspruch 1, 2 oder 3, worin $R^2$ Methyl ist, substituiert durch Carboxy, Cyano oder Halogen.

7. Verbindung nach Anspruch 1, 2 oder 3 worin $R^2$ Methyl ist, substituiert durch Phenyl, Thienyl, Pyridyl, Imidazolyl, Thiazolyl oder Morpholino, das weiter substituiert sein kann durch $C_{1-20}$ Alkyl, $C_{1-3}$ Alkyl, substituiert durch Hydroxy, Carboxyl, $C_{2-5}$ Alkoxycarbonyl, Piperazyl oder Phenylthio, $C_{2-4}$ Alkenyl, Hydroxy, Halogen, Nitro, Formyl, $C_{1-3}$ Alkoxy, Carboxy, Trifluormethyl, Di-$C_{1-3}$ Alkylamino, $C_{5-7}$ Cycloalkyl oder $C_{1-3}$ Alkylthio.

8. Verbindung nach Anspruch 1, 2 oder 3, worin $R^2$ $C_{2-15}$ Alkyl oder $C_{2-15}$ Alkenyl ist, das substituiert sein kann durch Carboxy, $C_{2-5}$ Alkoxycarbonyl oder Phenyl, das substituiert sein kann durch Halogen, $C_{1-3}$ Alkyl, $C_{1-3}$ Alkoxy oder Hydroxy.

9. Verbindung nach Anspruch 1, 2 oder 3 worin $R^4$ Wasserstoff oder $C_{1-10}$ Alkanoyl ist.

10. Verbindung nach Anspruch 1, 2 oder 3, worin $R^5$ und $R^6$ jeweils $C_{1-6}$ Alkyl oder $C_{1-3}$ Alkoxy sind.

11. Verbindung nach Anspruch 1, 2 oder 3, worin $R^1$ Methyl ist; $R^2$ $C_{2-6}$ Alkyl oder $C_{2-6}$ Alkenyl, das substituiert ist durch Phenyl, das substituiert sein kann durch $C_{1-3}$ Alkyl, $C_{1-3}$ Alkoxy oder Halogen; $R^3$ Methyl ist; $R^4$ Wasserstoff ist; und $R^5$ und $R^6$ jeweils Methyl bedeuten.

12. Verbindung nach Anspruch 1, 2 oder 3, die 5-Hydroxy-2-cinnamoyl-2,4,6,7-tetramethyl-2,3-dihydrobenzofuran ist.

13. Verbindung nach Anspruch 1, 2, oder 3, die 5-Hydroxy-2-phenylethyl-2,4,6,7-tetramethyl-2,3-dihydrobenzofuran ist.

14. Verbindung nach Anspruch 1, 2 oder 3, die 5-Hydroxy-2-(1-(Z)-pentenyl)-2,4,6,7-tetramethyl-2,3-dihydrobenzofuran ist.

15. Verwendung einer Verbindung nach Anspruch 1, 2 oder 3 oder eines Salzes davon zur Herstellung eines 5-Lipoxygenase- und/oder 12-Lipoxygenaseinhibitors.

16. Verwendung einer Verbindung der Formel:

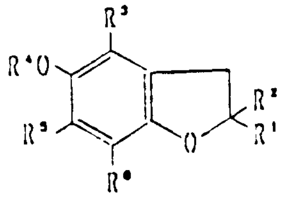

(I)

worin $R^1$ Wasserstoff oder $C_{1-6}$ Alkyl ist; $R^2$ Methyl ist, das durch Carboxy, $C_{2-5}$ Alkoxycarbonyl, Cyano, Halogen, Phenyl, 1-Naphthyl, 2-Naphtyl, Indanyl, Tetralyl oder eine heterocyclische Gruppe, ausgewählt aus Thienyl, Pyridyl, Imidazolyl, Thiazolyl, Morpholino und Thiomorpholino substituiert ist, wobei die Phenyl- oder heterocyclische Gruppe gegebenenfalls weiter substituiert ist mit einem oder mehreren Substituenten, ausgewählt aus $C_{1-20}$ Alkyl, $C_{1-6}$ Alkyl, substituiert durch Hydroxyl, Carboxyl, $C_{2-5}$ Alkoxycarbonyl, Piperazyl oder Phenylthio, $C_{2-4}$ Alkenyl, Halogen, Nitro, Formyl, $C_{1-3}$ Alkoxy, Carboxy, Trifluormethyl, Di-$C_{1-3}$ Alkylamino, $C_{5-7}$ Cycloalkyl und $C_{1-3}$ Alkylthio; oder $R^2$ eine $C_{2-15}$ Alkyl- oder $C_{2-15}$ Alkenylgruppe bedeutet, die substituiert sein kann durch Carboxy, $C_{2-5}$ Alkoxycarbonyl, Halogen, Cyano, Phenyl, 1-Naphthyl, 2-Naphthyl, Indanyl, Tetralyl oder eine heterocyclische Gruppe ausgewählt aus Thienyl, Pyridyl, Imidazolyl, Thiazolyl, Morpholino und Thiomorpholino, wobei die Phenyl- oder heterocyclische Gruppe gegebenenfalls weiter substituiert sein kann durch Halogen, $C_{1-3}$ Alkyl, $C_{1-3}$ Alkoxy oder Hydroxy, mit der Maßgabe, daß die Alkyl- oder Alkenylgruppe nicht substituiert ist an dem Kohlenstoffatom in der alpha-Position zum Benzofuranring; $R^3$ $C_{1-6}$ Alkyl ist; $R^4$ Wasserstoff, $C_{1-10}$ Alkanoyl oder cyclisches $C_{3-10}$ Alkanoyl ist; $R^5$ und $R^6$ jeweils $C_{1-6}$ Alkyl oder $C_{1-3}$ Alkoxy bedeuten, oder $R^5$ und $R^6$ zusammengenommen Butadienylen bedeuten, wobei ein kondensierter Benzolring gebildet wird, der substituiert sein kann durch 1 bis 3 Substituenten, ausgewählt aus der Gruppe, die besteht aus $C_{1-3}$ Alkyl, $C_{1-3}$ Alkoxy, Hydroxyl, Nitro und Halogen, oder ein Salz davon, zur Herstellung eines Medikaments zur Verbesserung des Kreislaufsystems, zur Behandlung allergischer Krankheiten oder zur Behandlung des zentralen Nervensystems.

**17.** Verwendung nach Anspruch 16, worin das Medikament ein 5-Lipoxygenase und/oder 12-Lipoxygenaseinhibitor ist.

**18.** Verfahren zur Herstellung einer Verbindung nach Anspruch 1, umfassend das Umsetzen einer Verbindung der Formel:

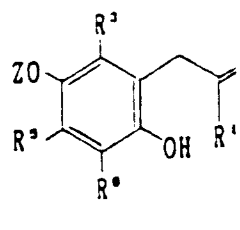

(II)

worin $R^1$, $R^3$ $R^5$ und $R^6$ die gleiche Bedeutung wie oben angegeben haben, Z Wasserstoff ist oder eine Hydroxylschutzgruppe, mit einem Halogenmolekül in Gegenwart einer Base, um einen Ringschluß zu bewirken, oder durch Unterwerfen der Verbindung (II) der Behandlung mit einer Persäure in Gegenwart einer Base, um einen Ringschluß zu bewirken, gefolgt davon, daß man die so ringgeschlossene Verbindung im Falle des 2-Hydroxymethylderivats mit einem Oxidationsmittel reagieren läßt, die so erhaltene Verbindung dann einer Additions-Eliminierungsreaktion mit einer Verbindung unterwirft, dargestellt durch die Formel

$$(C_6H_5)_3P^{\oplus}\text{-}R^{2'}X^{\ominus} \qquad \text{(III)}$$

worin X Halogen ist, $R^{2'}$ ein Kohlenwasserstoffrest, dessen Kohlenstoffanzahl um eins geringer als die von $R^2$ ist, gefolgt von, wenn gewünscht, Unterwerfen des erhaltenen Produkts der Entfernung der Schutzgruppe, Acylierung, Hydrierung oder(und) der Substituenten-Austausch-Reaktion.

**Patentansprüche für folgende Vertragsstaaten : ES, GR**

**1.** Verfahren zur Herstellung einer Verbindung der Formel:

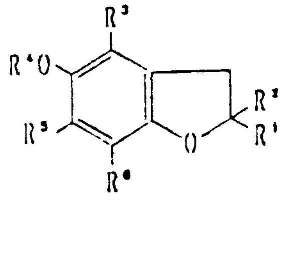

(I)

worin $R^1$ Wasserstoff oder $C_{1-6}$ Alkyl ist; $R^2$ Methyl ist, das durch Carboxy, $C_{2-5}$ Alkoxycarbonyl, Cyano, Halogen, Phenyl, 1-Naphthyl, 2-Naphtyl, Indanyl, Tetralyl oder eine heterocyclische Gruppe, ausgewählt aus Thienyl, Pyridyl, Imidazolyl, Thiazolyl, Morpholino und Thiomorpholino substituiert ist, wobei die Phenyl- oder heterocyclische Gruppe gegebenenfalls weiter substituiert ist mit einem oder mehreren Substituenten, ausgewählt aus $C_{1-20}$ Alkyl, $C_{1-6}$ Alkyl, substituiert durch Hydroxyl, Carboxyl, $C_{2-5}$ Alkoxycarbonyl, Piperazyl oder Phenylthio, $C_{2-4}$ Alkenyl, Halogen, Nitro, Formyl, $C_{1-3}$ Alkoxy, Carboxy, Trifluormethyl, Di-$C_{1-3}$ Alkylamino, $C_{5-7}$ Cycloalkyl und $C_{1-3}$ Alkylthio; oder $R^2$ eine $C_{2-15}$ Alkyl- oder $C_{2-15}$ Alkenylgruppe bedeutet, die substituiert sein kann durch Carboxy, $C_{2-5}$ Alkoxycarbonyl, Halogen, Cyano, Phenyl, 1-Naphthyl, 2-Naphthyl, Indanyl, Tetralyl oder eine heterocyclische Gruppe ausgewählt aus Thienyl, Pyridyl, Imidazolyl, Thiazolyl, Morpholino und Thiomorpholino, wobei die Phenyl- oder heterocyclische Gruppe gegebenenfalls weiter substituiert sein kann durch Halogen, $C_{1-3}$ Alkyl, $C_{1-3}$ Alkoxy oder Hydroxy, mit der Maßgabe, daß die Alkyl- oder Alkenylgruppe nicht substituiert ist an dem Kohlenstoffatom in der alpha-Position zum Benzofuranring; $R^3$ $C_{1-6}$ Alkyl ist; $R^4$ Wasserstoff, $C_{1-10}$ Alkanoyl oder cyclisches $C_{3-10}$ Alkanoyl ist; $R^5$ und $R^6$ jeweils $C_{1-6}$ Alkyl oder $C_{1-3}$ Alkoxy bedeuten, oder $R^5$ und $R^6$ zusammengenommen Butadienylen bedeuten, wobei ein kondensierter Benzolring gebildet wird, der substituiert sein kann durch 1 bis 3 Substituenten, ausgewählt aus der Gruppe, die besteht aus $C_{1-3}$ Alkyl, $C_{1-3}$ Alkoxy, Hydroxyl, Nitro und Halogen, oder eines Salzes davon, mit Ausnahme von 5-Hydroxy-4,6,7-trimethyl-2-isopropyl-2,3-dihydrobenzofuran, 5-Acetoxy-4,6,7-trimethyl-2-isopropyl-2,3-dihydrobenzofuran, 5-Hydroxy-4,6,7-trimethyl-2-n-propyl-2,3-dihydrobenzofuran, 5-Hydroxy-4,6,7-trimethyl-2-ethyl-2,3-dihydrobenzofuran, 5-Hydroxy-4,6,7-trimethyl-2-n-tridecyl-2,3-dihydrobenzofuran, und 5-Hydroxy-2,4,6,7-tetramethyl-2-(3',7',11'-trimethyl-dodecyl)-2,3-dihydrobenzofuran, das umfaßt das Umsetzen einer Verbindung der Formel:

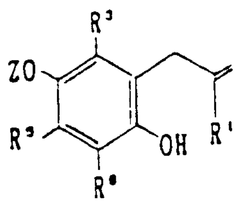

(II)

worin $R^1$, $R^3$, $R^5$ und $R^6$ die gleiche Bedeutung wie oben angegeben haben, Z Wasserstoff ist oder eine Hydroxylschutzgruppe, mit einem Halogenmolekül in Gegenwart einer Base, um einen Ringschluß zu bewirken, oder durch Unterwerfen der Verbindung (II) der Behandlung mit einer Persäure in Gegenwart einer Base, um einen Ringschluß zu bewirken, gefolgt davon, daß man die so ringgeschlossene Verbindung im Falle des 2-Hydroxymethylderivats mit einem Oxidationsmittel reagieren läßt, die so erhaltene Verbindung dann einer Additions-Eliminierungsreaktion mit einer Verbindung unterwirft, dargestellt durch die Formel

$$(C_6H_5)_3P^{\oplus}\text{-}R^{2'}X^{\ominus} \qquad \text{(III)}$$

worin X Halogen ist, $R^{2'}$ ein Kohlenwasserstoffrest ist, dessen Kohlenstoffanzahl um eins geringer als die von $R^2$ ist, gefolgt von, wenn gewünscht, Unterwerfen des erhaltenen Produkts der Entfernung der Schutzgruppe, Acylierung, Hydrierung oder(und) der Substituenten-Austausch-Reaktion.

2. Verfahren zur Herstellung eines Medikaments zur Verbesserung des Kreislaufsystems, zur Behandlung des zentralen Nervensystems oder zur Behandlung allergischer Krankheiten, umfassend das Mischen einer Menge, die wirksam 5-Lipoxygenase und/oder 12-Lipoxygenase inhibiert, einer Verbindung der Formel:

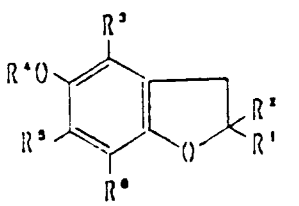

(I)

worin $R^1$ Wasserstoff oder $C_{1-6}$ Alkyl ist; $R^2$ Methyl ist, das durch Carboxy, $C_{2-5}$ Alkoxycarbonyl, Cyano, Halogen,

Phenyl, 1-Naphthyl, 2-Naphtyl, Indanyl, Tetralyl oder eine heterocyclische Gruppe, ausgewählt aus Thienyl, Pyridyl, Imidazolyl, Thiazolyl, Morpholino und Thiomorpholino substituiert ist, wobei die Phenyl- oder heterocyclische Gruppe gegebenenfalls weiter substituiert ist mit einem oder mehreren Substituenten, ausgewählt aus $C_{1-20}$ Alkyl, $C_{1-6}$ Alkyl, substituiert durch Hydroxyl, Carboxyl, $C_{2-5}$ Alkoxycarbonyl, Piperazyl oder Phenylthio, $C_{2-4}$ Alkenyl, Halogen, Nitro, Formyl, $C_{1-3}$ Alkoxy, Carboxy, Trifluormethyl, Di-$C_{1-3}$ Alkylamino, $C_{5-7}$ Cycloalkyl und $C_{1-3}$ Alkylthio; oder $R^2$ eine $C_{2-15}$ Alkyl- oder $C_{2-15}$ Alkenylgruppe bedeutet, die substituiert sein kann durch Carboxy, $C_{2-5}$ Alkoxycarbonyl, Halogen, Cyano, Phenyl, 1-Naphthyl, 2-Naphthyl, Indanyl, Tetralyl oder eine heterocyclische Gruppe ausgewählt aus Thienyl, Pyridyl, Imidazolyl, Thiazolyl, Morpholino und Thiomorpholino, wobei die Phenyl- oder heterocyclische Gruppe gegebenenfalls weiter substituiert sein kann durch Halogen, $C_{1-3}$ Alkyl, $C_{1-3}$ Alkoxy oder Hydroxy, mit der Maßgabe, daß die Alkyl- oder Alkenylgruppe nicht substituiert ist an dem Kohlenstoffatom in der alpha-Position zum Benzofuranring; $R^3$ $C_{1-6}$ Alkyl ist; $R^4$ Wasserstoff, $C_{1-10}$ Alkanoyl oder cyclisches $C_{3-10}$ Alkanoyl ist; $R^5$ und $R^6$ jeweils $C_{1-6}$ Alkyl oder $C_{1-3}$ Alkoxy bedeuten, oder $R^5$ und $R^6$ zusammengenommen Butadienylen bedeuten, wobei ein kondensierter Benzolring gebildet wird, der substituiert sein kann durch 1 bis 3 Substituenten, ausgewählt aus der Gruppe, die besteht aus $C_{1-3}$ Alkyl, $C_{1-3}$ Alkoxy, Hydroxyl, Nitro und Halogen, oder eines Salzes davon, mit Ausnahme von 5-Hydroxy-2,4,6,7-tetramethyl-2-(3',7',11'-trimethyl-dodecyl)-2,3-dihydrobenzofuran, mit einem per se bekannten pharmazeutisch annehmbaren Träger oder Excipienten.

**3.** Verfahren nach Anspruch 2, worin das Medikament ein 5-Lipoxygenase- und/oder 12-Lipoxygenaseinhibitor ist.

**4.** Verfahren nach Anspruch 1, 2 oder 3, worin $R^1$ Wasserstoff oder $C_{1-3}$ Alkyl ist.

**5.** Verfahren nach Anspruch 1, 2 oder 3, worin $R^2$ Methyl ist, substituiert durch Carboxy, Cyano oder Halogen.

**6.** Verfahren nach Anspruch 1, 2 oder 3, worin $R^2$ Methyl ist, substituiert durch Phenyl, Thienyl, Pyridyl, Imidazolyl, Thiazolyl oder Morpholino, das weiter substituiert sein kann durch $C_{1-20}$ Alkyl, $C_{1-3}$ Alkyl, substituiert durch Hydroxy, Carboxyl, $C_{2-5}$ Alkoxycarbonyl, Piperazyl oder Phenylthio, $C_{2-4}$ Alkenyl, Hydroxy, Halogen, Nitro, Formyl, $C_{1-3}$ Alkoxy, Carboxy, Trifluormethyl, Di-$C_{1-3}$ Alkylamino, $C_{5-7}$ Cycloalkyl oder $C_{1-3}$ Alkylthio.

**7.** Verfahren nach Anspruch 1, 2 oder 3, worin $R^2$ $C_{2-15}$ Alkyl oder $C_{2-15}$ Alkenyl ist, das substituiert sein kann durch Carboxy, $C_{2-5}$ Alkoxycarbonyl oder Phenyl, das substituiert sein kann durch Halogen, $C_{1-3}$ Alkyl, $C_{1-3}$ Alkoxy oder Hydroxy.

**8.** Verfahren nach Anspruch 1, 2 oder 3, worin $R^4$ Wasserstoff oder $C_{1-10}$ Alkanoyl ist.

**9.** Verfahren nach Anspruch 1, 2 oder 3, worin $R^5$ und $R^6$ jeweils $C_{1-6}$ Alkyl oder $C_{1-3}$ Alkoxy sind.

**10.** Verfahren nach Anspruch 1, 2 oder 3, worin $R^1$ Methyl ist; $R^2$ $C_{2-6}$ Alkyl oder $C_{2-6}$ Alkenyl ist, das substituiert ist durch Phenyl, das substituiert sein kann durch $C_{1-3}$ Alkyl, $C_{1-3}$ Alkoxy oder Halogen; $R^3$ Methyl ist; $R^4$ Wasserstoff ist; und $R^5$ und $R^6$ jeweils Methyl bedeuten.

**11.** Verfahren nach Anspruch 1, 2 oder 3, worin die Verbindung 5-Hydroxy-2-cinnamoyl-2,4,6,7-tetramethyl-2,3-dihydrobenzofuran ist.

**12.** Verfahren nach Anspruch 1, 2 oder 3, worin die Verbindung 5-Hydroxy-2-phenylethyl-2,4,6,7-tetramethyl-2,3-dihydrobenzofuran ist.

**13.** Verfahren nach Anspruch 1, 2 oder 3, worin die Verbindung 5-Hydroxy-2-(1-(Z)-pentenyl)-2,4,6,7-tetramethyl-2,3-dihydrobenzofuran ist.

**14.** Verwendung einer Verbindung nach Anspruch 2 oder eines Salzes davon oder von 5-Hydroxy-2,4,6,7-tetramethyl-2-(3',7',11'-trimethyl-dodecyl)-2,3-dihydrobenzofuran als Komponente bei der Herstellung eines Medikaments zur Inhibierung von 5-Lipoxygenase und/oder 12-Lipoxygenase.

**Revendications**

**Revendications pour les Etats contractants suivants : AT, BE, CH, DE, FR, GB, IT, LI, LU, NL, SE**

**1.** Composé de formule :

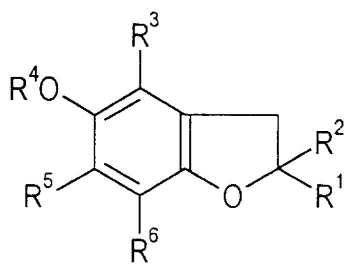

(I)

dans laquelle $R^1$ représente un atome d'hydrogène ou un groupe alkyle en $C_1$-$C_6$ ; $R^2$ représente un groupe méthyle qui est substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou plusieurs substituants choisis parmi alkyle en $C_1$-$C_{20}$, alkyle en $C_1$-$C_6$ substitué par hydroxy, carboxy, (alcoxy en $C_2$-$C_5$)-carbonyle, pipérazyle ou phénylthio, alcényle en $C_2$-$C_4$, halogéno, nitro, formyle, alcoxy en $C_1$-$C_3$, carboxy, trifluorométhyle, di(alkyle en $C_1$-$C_3$)amino, cycloalkyle en $C_5$-$C_7$ et alkylthio en $C_1$-$C_3$ ; ou bien $R^2$ représente un groupe alkyle en $C_2$-$C_{15}$ ou alcényle en $C_2$-$C_{15}$ qui peut être substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou des atomes d'halogène ou par un ou des groupes alkyle en $C_1$-$C_3$, alcoxy en $C_1$-$C_3$ ou hydroxy, à condition que ledit groupe alkyle ou alcényle ne soit pas substitué au niveau de l'atome de carbone situé en position $\alpha$ par rapport au noyau benzofuranne ; $R^3$ représente un groupe alkyle en $C_1$-$C_6$ ; $R^4$ représente un atome d'hydrogène, un groupe alcanoyle en $C_1$-$C_{10}$ ou un groupe alcanoyle cyclique en $C_3$-$C_{10}$; $R^5$ et $R^6$ représentent chacun un groupe alkyle en $C_1$-$C_6$ ou alcoxy en $C_1$-$C_3$, ou bien $R^5$ et $R^6$ représentent conjointement un groupe butadiénylène, pour former un noyau benzénique condensé qui peut être substitué par de 1 à 3 substituants choisis dans le groupe constitué par les atomes d'halogène et les groupes alkyle en $C_1$-$C_3$, alcoxy en $C_1$-$C_3$, hydroxy et nitro ;
ou un sel d'un tel composé, à l'exclusion des 5-hydroxy-4,6,7-triméthyl-2-isopropyl-2,3-dihydrobenzofuranne, 5-acétoxy-4,6,7-triméthyl-2-isopropyl-2,3-dihydrobenzofuranne, 5-hydroxy-4,6,7-triméthyl-2-n-propyl-2,3-dihydrobenzofuranne, 5-hydroxy-4,6,7-triméthyl-2-éthyl-2,3-dihydrobenzofuranne, 5-hydroxy-4,6,7-triméthyl-2-n-tridécyl-2,3-dihydrobenzofuranne, et 5-hydroxy-2,4,6,7-tétraméthyl-2-(3',7',11'-triméthyl-dodécyl)-2,3-dihydrobenzofuranne.

2. Composé de formule :

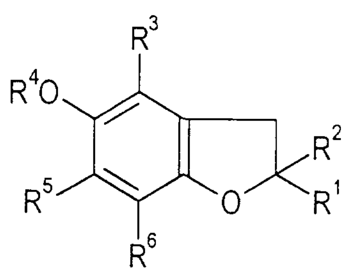

(I)

dans laquelle $R^1$ représente un atome d'hydrogène ou un groupe alkyle en $C_1$-$C_6$ ; $R^2$ représente un groupe méthyle qui est substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou plusieurs substituants choisis parmi alkyle en $C_1$-$C_{20}$, alkyle en $C_1$-$C_6$ substitué par hydroxy, carboxy, (alcoxy en $C_2$-$C_5$)carbonyle, pipérazyle ou phénylthio, alcényle en $C_2$-$C_4$, halogéno, nitro, formyle, alcoxy en $C_1$-$C_3$, carboxy, trifluorométhyle, di(alkyle en $C_1$-$C_3$)amino, cycloalkyle en $C_5$-$C_7$ et alkylthio en $C_1$-$C_3$ ; ou bien $R^2$ représente un groupe alkyle en $C_2$-$C_{15}$ ou alcényle en $C_2$-$C_{15}$ qui peut être substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle,

pyridyle, imidazolyle, thiazolyle, morpholino et thiomorpholino, ledit groupe phényle ou hétérocyclique étant éventuellement encore substitué par un ou des atomes d'halogène ou par un ou des groupes alkyle en $C_1$-$C_3$, alcoxy en $C_1$-$C_3$ ou hydroxy, à condition que ledit groupe alkyle ou alcényle ne soit pas substitué au niveau de l'atome de carbone situé en position $\alpha$ par rapport au noyau benzofuranne ; $R^3$ représente un groupe alkyle en $C_1$-$C_6$ ; $R^4$ représente un atome d'hydrogène, un groupe alcanoyle en $C_1$-$C_{10}$ ou un groupe alcanoyle cyclique en $C_3$-$C_{10}$ ; $R^5$ et $R^6$ représentent chacun un groupe alkyle en $C_1$-$C_6$ ou alcoxy en $C_1$-$C_3$, ou bien $R^5$ et $R^6$ représentent conjointement un groupe butadiénylène, pour former un noyau benzénique condensé qui peut être substitué par de 1 à 3 substituants choisis dans le groupe constitué par les atomes d'halogène et les groupes alkyle en $C_1$-$C_3$, alcoxy en $C_1$-$C_3$, hydroxy et nitro ;
ou un sel d'un tel composé, à l'exclusion du 5-hydroxy-2,4,6,7-tétraméthyl-2-(3',7',11'-triméthyl-dodécyl)-2,3-dihydrobenzofuranne, comme médicament en vue d'un traitement thérapeutique chez l'homme ou chez un animal.

3. Composé conforme à la revendication 2, le médicament étant destiné à améliorer le système circulatoire, à traiter le système nerveux central ou à traiter des maladies allergiques.

4. Composé conforme à la revendication 3, comme inhibiteur de 5-lipoxygénase et/ou de 12-lipoxygénase.

5. Composé conforme à la revendication 1, 2 ou 3, dans lequel $R^1$ représente un atome d'hydrogène ou un groupe alkyle en $C_1$-$C_3$.

6. Composé conforme à la revendication 1, 2 ou 3, dans lequel $R^2$ représente un groupe méthyle substitué par un atome d'halogène ou par un groupe cyano ou carboxy.

7. Composé conforme à la revendication 1, 2 ou 3, dans lequel $R^2$ représente un groupe méthyle substitué par un groupe phényle, thiényle, pyridyle, imidazolyle, thiazolyle ou morpholino, lequel peut être encore substitué par un ou des atomes d'halogène ou par un ou des groupes alkyle en $C_1$-$C_{20}$, alkyle en $C_1$-$C_3$ substitué par hydroxy, carboxy, (alcoxy en $C_2$-$C_5$)carbonyle, pipérazyle ou phénylthio, alcényle en $C_2$-$C_4$, hydroxy, nitro, formyle, alcoxy en $C_1$-$C_3$, carboxy, trifluorométhyle, di(alkyl en $C_1$-$C_3$)amino, cycloalkyle en $C_5$-$C_7$ ou alkylthio en $C_1$-$C_3$.

8. Composé conforme à la revendication 1, 2 ou 3, dans lequel $R^2$ représente un groupe alkyle en $C_2$-$C_{15}$ ou alcényle en $C_2$-$C_{15}$ qui peut être substitué par un ou des groupes carboxy, (alcoxy en $C_2$-$C_5$)-carbonyle ou phényle éventuellement substitué par un ou des atomes d'halogène ou par un ou des groupes alkyle en $C_1$-$C_3$, alcoxy en $C_1$-$C_3$ ou hydroxy.

9. Composé conforme à la revendication 1, 2 ou 3, dans lequel $R^4$ représente un atome d'hydrogène ou un groupe alcanoyle en $C_1$-$C_{10}$.

10. Composé conforme à la revendication 1, 2 ou 3, dans lequel $R^5$ et $R^6$ représentent chacun un groupe alkyle en $C_1$-$C_6$ ou un groupe alcoxy en $C_1$-$C_3$.

11. Composé conforme à la revendication 1, 2 ou 3, dans lequel $R^1$ représente un groupe méthyle ; $R^2$ représente un groupe alkyle en $C_2$-$C_6$ ou alcényle en $C_2$-$C_6$, substitué par un groupe phényle éventuellement substitué par un ou des atomes d'halogène ou par un ou des groupes alkyle en $C_1$-$C_3$ ou alcoxy en $C_1$-$C_3$ ; $R^3$ représente un groupe méthyle ; $R^4$ représente un atome d'hydrogène ; et $R^5$ et $R^6$ représentent chacun un groupe méthyle.

12. Composé conforme à la revendication 1, 2 ou 3, qui est le 5-hydroxy-2-cinnamoyl-2,4,6,7-tétraméthyl-2,3-dihydrobenzofuranne.

13. Composé conforme à la revendication 1, 2 ou 3, qui est le 5-hydroxy-2-phényléthyl-2,4,6,7-tétraméthyl-2,3-dihydrobenzofuranne.

14. Composé conforme à la revendication 1, 2 ou 3, qui est le 5-hydroxy-2-(1-(Z)-penténycl)-2,4,6,7-tétraméthyl-2,3-dihydrobenzofuranne.

15. Utilisation d'un composé conforme à la revendication 1, 2 ou 3, ou d'un sel d'un tel composé, pour la préparation d'un inhibiteur de 5-lipoxygénase et/ou de 12-lipoxygénase.

16. Utilisation d'un composé de formule :

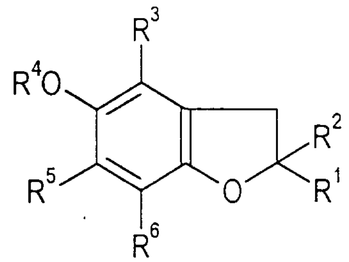

(I)

dans laquelle $R^1$ représente un atome d'hydrogène ou un groupe alkyle en $C_1$-$C_6$ ; $R^2$ représente un groupe méthyle qui est substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou plusieurs substituants choisis parmi alkyle en $C_1$-$C_{20}$, alkyle en $C_1$-$C_6$ substitué par hydroxy, carboxy, (alcoxy en $C_2$-$C_5$)-carbonyle, pipérazyle ou phénylthio, alcényle en $C_2$-$C_4$, halogéno, nitro, formyle, alcoxy en $C_1$-$C_3$, carboxy, trifluorométhyle, di(alkyle en $C_1$-$C_3$)amino, cycloalkyle en $C_5$-$C_7$ et alkylthio en $C_1$-$C_3$ ; ou bien $R^2$ représente un groupe alkyle en $C_2$-$C_{15}$ ou alcényle en $C_2$-$C_{15}$ qui peut être substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou des atomes d'halogène ou par un ou des groupes alkyle en $C_1$-$C_3$, alcoxy en $C_1$-$C_3$ ou hydroxy, à condition que ledit groupe alkyle ou alcényle ne soit pas substitué au niveau de l'atome de carbone situé en position $\alpha$ par rapport au noyau benzofuranne ; $R^3$ représente un groupe alkyle en $C_1$-$C_6$ ; $R^4$ représente un atome d'hydrogène, un groupe alcanoyle en $C_1$-$C_{10}$ ou un groupe alcanoyle cyclique en $C_3$-$C_{10}$; $R^5$ et $R^6$ représentent chacun un groupe alkyle en $C_1$-$C_6$ ou alcoxy en $C_1$-$C_3$, ou bien $R^5$ et $R^6$ représentent conjointement un groupe butadiénylène, pour former un noyau benzénique condensé qui peut être substitué par de 1 à 3 substituants choisis dans le groupe constitué par les atomes d'halogène et les groupes alkyle en $C_1$-$C_3$, alcoxy en $C_1$-$C_3$, hydroxy et nitro ;
ou d'un sel d'un tel composé, pour préparer un médicament destiné à améliorer le système circulatoire, à traiter des maladies allergiques ou à traiter le système nerveux central.

**17.** Utilisation conforme à la revendication 16, dans laquelle le médicament est un inhibiteur de 5-lipoxygénase et/ou de 12-lipoxygénase.

**18.** Procédé de production d'un composé conforme à la revendication 1, qui comporte la réaction d'un composé de formule :

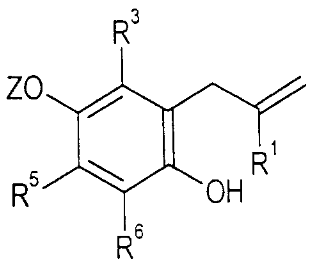

(II)

dans laquelle $R^1$, $R^3$, $R^5$ et $R^6$ ont les mêmes significations que celles définies plus haut et Z représente un atome d'hydrogène ou un groupe hydroxy-protecteur,
avec une molécule d'halogène, en présence d'une base, de façon à provoquer une fermeture de cycle, ou la soumission du composé (II) à un traitement avec un peracide, en présence d'une base, de façon à provoquer une fermeture de cycle, suivie, dans le cas d'un dérivé 2-hydroxyméthylé, de la réaction du composé à chaîne ainsi cyclisée avec un agent oxydant, puis la soumission du composé ainsi obtenu à une réaction d'additionélimination avec un composé représenté par la formule :

$$(C_6H_5)_3P^{+}\text{-}R^{2'}\ X^{-} \qquad (III)$$

dans laquelle X représente un atome d'halogène, et $R^{2'}$ est un résidu hydrocarboné dont le nombre d'atomes de carbone est inférieur d'une unité à celui de $R^2$,
puis, si on le souhaite, la soumission du produit résultant à une réaction de déprotection, d'acylation, d'hydrogénation et/ou d'échange de substituants, respectivement.

**Revendications pour les Etats contractants suivants : ES, GR**

**1.** Procédé de production d'un composé de formule :

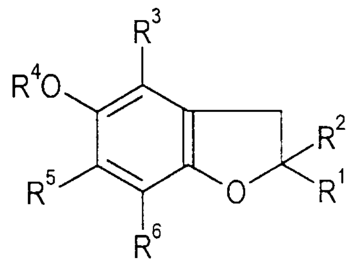

(I)

dans laquelle $R^1$ représente un atome d'hydrogène ou un groupe alkyle en $C_1$-$C_6$ ; $R^2$ représente un groupe méthyle qui est substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou plusieurs substituants choisis parmi alkyle en $C_1$-$C_{20}$, alkyle en $C_1$-$C_6$ substitué par hydroxy, carboxy, (alcoxy en $C_2$-$C_5$)-carbonyle, pipérazyle ou phénylthio, alcényle en $C_2$-$C_4$, halogéno, nitro, formyle, alcoxy en $C_1$-$C_3$, carboxy, trifluorométhyle, di(alkyle en $C_1$-$C_3$)amino, cycloalkyle en $C_5$-$C_7$ et alkylthio en $C_1$-$C_3$ ; ou bien $R^2$ représente un groupe alkyle en $C_2$-$C_{15}$ ou alcényle en $C_2$-$C_{15}$ qui peut être substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou des atomes d'halogène ou par un ou des groupes alkyle en $C_1$-$C_3$, alcoxy en $C_1$-$C_3$ ou hydroxy, à condition que ledit groupe alkyle ou alcényle ne soit pas substitué au niveau de l'atome de carbone situé en position $\alpha$ par rapport au noyau benzofuranne ; $R^3$ représente un groupe alkyle en $C_1$-$C_6$ ; $R^4$ représente un atome d'hydrogène, un groupe alcanoyle en $C_1$-$C_{10}$ ou un groupe alcanoyle cyclique en $C_3$-$C_{10}$; $R^5$ et $R^6$ représentent chacun un groupe alkyle en $C_1$-$C_6$ ou alcoxy en $C_1$-$C_3$, ou bien $R^5$ et $R^6$ représentent conjointement un groupe butadiénylène, pour former un noyau benzénique condensé qui peut être substitué par de 1 à 3 substituants choisis dans le groupe constitué par les atomes d'halogène et les groupes alkyle en $C_1$-$C_3$, alcoxy en $C_1$-$C_3$, hydroxy et nitro ;
ou un sel d'un tel composé, à l'exclusion des 5-hydroxy-4,6,7-triméthyl-2-isopropyl-2,3-dihydrobenzofuranne, 5-acétoxy-4,6,7-triméthyl-2-isopropyl-2,3-dihydrobenzofuranne, 5-hydroxy-4,6,7-triméthyl-2-n-propyl-2,3-dihydrobenzofuranne, 5-hydroxy-4,6,7-triméthyl-2-éthyl-2,3-dihydrobenzofuranne, 5-hydroxy-4,6,7-triméthyl-2-n-tridécyl-2,3-dihydrobenzofuranne, et 5-hydroxy-2,4,6,7-tétraméthyl-2-(3',7',11'-triméthyl-dodécyl)-2,3-dihydrobenzofuranne,
lequel procédé comporte la réaction d'un composé de formule :

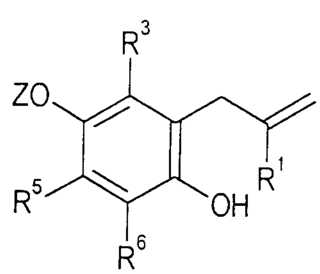

(II)

dans laquelle $R^1$, $R^3$, $R^5$ et $R^6$ ont les mêmes significations que celles définies plus haut et Z représente un atome d'hydrogène ou un groupe hydroxy-protecteur,
avec une molécule d'halogène, en présence d'une base, de façon à provoquer une fermeture de cycle, ou la soumission du composé (II) à un traitement avec un peracide, en présence d'une base, de façon à provoquer une fermeture de cycle, suivie, dans le cas d'un dérivé 2-hydroxyméthylé, de la réaction du composé à chaîne ainsi cyclisée avec un agent oxydant, puis la soumission du composé ainsi obtenu à une réaction d'addition-élimination avec un composé représenté par la formule :

$$(C_6H_5)_3P^+\text{-}R^{2'}\ X^- \qquad \text{(III)}$$

dans laquelle X représente un atome d'halogène, et $R^{2'}$ est un résidu hydrocarboné dont le nombre d'atomes de carbone est inférieur d'une unité à celui de $R^2$,
puis, si on le souhaite, la soumission du produit résultant à une réaction de déprotection, d'acylation, d'hydrogénation et/ou d'échange de substituants, respectivement.

**2.** Procédé de production d'un médicament destiné à améliorer le système circulatoire, à traiter le système nerveux central ou à traiter des maladies allergiques, qui comporte le fait de mélanger une certaine quantité, suffisante pour inhiber effectivement la 5-lipoxygénase et/ou la 12-lipoxygénase, d'un composé de formule :

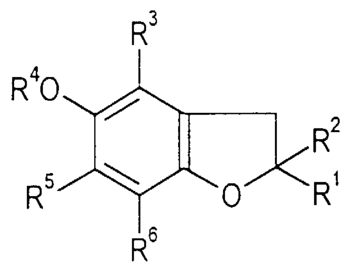

(I)

dans laquelle $R^1$ représente un atome d'hydrogène ou un groupe alkyle en $C_1$-$C_6$ ; $R^2$ représente un groupe méthyle qui est substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou plusieurs substituants choisis parmi alkyle en $C_1$-$C_{20}$, alkyle en $C_1$-$C_6$ substitué par hydroxy, carboxy, (alcoxy en $C_2$-$C_5$)-carbonyle, pipérazyle ou phénylthio, alcényle en $C_2$-$C_4$, halogéno, nitro, formyle, alcoxy en $C_1$-$C_3$, carboxy, trifluorométhyle, di(alkyle en $C_1$-$C_3$)amino, cycloalkyle en $C_5$-$C_7$ et alkylthio en $C_1$-$C_3$ ; ou bien $R^2$ représente un groupe alkyle en $C_2$-$C_{15}$ ou alcényle en $C_2$-$C_{15}$ qui peut être substitué par un ou des atomes d'halogène ou par un ou des groupes carboxy, (alcoxy en $C_2$-$C_5$)-carbonyle, cyano, phényle, 1-naphtyle, 2-naphtyle, indanyle ou tétralinyle, ou par un groupe hétérocyclique choisi parmi thiényle, pyridyle, imidazolyle, thiazolyle, morpholino et thiomorpholino, ledit groupe phényle ou hétérocyclique étant éventuellement encore substitué par un ou des atomes d'halogène ou par un ou des groupes alkyle en $C_1$-$C_3$, alcoxy en $C_1$-$C_3$ ou hydroxy, à condition que ledit groupe alkyle ou alcényle ne soit pas substitué au niveau de l'atome de carbone situé en position $\alpha$ par rapport au noyau benzofuranne ; $R^3$ représente un groupe alkyle en $C_1$-$C_6$ ; $R^4$ représente un atome d'hydrogène, un groupe alcanoyle en $C_1$-$C_{10}$ ou un groupe alcanoyle cyclique en $C_3$-$C_{10}$; $R^5$ et $R^6$ représentent chacun un groupe alkyle en $C_1$-$C_6$ ou alcoxy en $C_1$-$C_3$, ou bien $R^5$ et $R^6$ représentent conjointement un groupe butadiénylène, pour former un noyau benzénique condensé qui peut être substitué par de 1 à 3 substituants choisis dans le groupe constitué par les atomes d'halogène et les groupes alkyle en $C_1$-$C_3$, alcoxy en $C_1$-$C_3$, hydroxy et nitro ;
à l'exclusion du 5-hydroxy-2,4,6,7-tétraméthyl-2-(3',7',11'-triméthyl-dodécyl)-2,3-dihydrobenzofuranne, avec un véhicule ou un excipient connu, admissible en pharmacie.

**3.** Procédé conforme à la revendication 2, dans lequel le médicament est un inhibiteur de 5-lipoxygénase et/ou de 12-lipoxygénase.

**4.** Procédé conforme à la revendication 1, 2 ou 3, dans lequel $R^1$ représente un atome d'hydrogène ou un groupe alkyle en $C_1$-$C_3$.

**5.** Procédé conforme à la revendication 1, 2 ou 3, dans lequel $R^2$ représente un groupe méthyle substitué par un

atome d'halogène ou par un groupe cyano ou carboxy.

6. Procédé conforme à la revendication 1, 2 ou 3, dans lequel $R^2$ représente un groupe méthyle substitué par un groupe phényle, thiényle, pyridyle, imidazolyle, thiazolyle ou morpholino, lequel peut être encore substitué par un ou des atomes d'halogène ou par un ou des groupes alkyle en $C_1$-$C_{20}$, alkyle en $C_1$-$C_3$ substitué par hydroxy, carboxy, (alcoxy en $C_2$-$C_5$)carbonyle, pipérazyle ou phénylthio, alcényle en $C_2$-$C_4$, hydroxy, nitro, formyle, alcoxy en $C_1$-$C_3$, carboxy, trifluorométhyle, di(alkyl en $C_1$-$C_3$)amino, cycloalkyle en $C_5$-$C_7$ ou alkylthio en $C_1$-$C_3$.

7. Procédé conforme à la revendication 1, 2 ou 3, dans lequel $R^2$ représente un groupe alkyle en $C_2$-$C_{15}$ ou alcényle en $C_2$-$C_{15}$ qui peut être substitué par un ou des groupes carboxy, (alcoxy en $C_2$-$C_5$)-carbonyle ou phényle éventuellement substitué par un ou des atomes d'halogène ou par un ou des groupes alkyle en $C_1$-$C_3$, alcoxy en $C_1$-$C_3$ ou hydroxy.

8. Procédé conforme à la revendication 1, 2 ou 3, dans lequel $R^4$ représente un atome d'hydrogène ou un groupe alcanoyle en $C_1$-$C_{10}$.

9. Procédé conforme à la revendication 1, 2 ou 3, dans lequel $R^5$ et $R^5$ représentent chacun un groupe alkyle en $C_1$-$C_6$ ou un groupe alcoxy en $C_1$-$C_3$.

10. Procédé conforme à la revendication 1, 2 ou 3, dans lequel $R^1$ représente un groupe méthyle ; $R^2$ représente un groupe alkyle en $C_2$-$C_6$ ou alcényle en $C_2$-$C_6$, substitué par un groupe phényle éventuellement substitué par un ou des atomes d'halogène ou par un ou des groupes alkyle en $C_1$-$C_3$ ou alcoxy en $C_1$-$C_3$ ; $R^3$ représente un groupe méthyle ; $R^4$ représente un atome d'hydrogène ; et $R^5$ et $R^6$ représentent chacun un groupe méthyle.

11. Procédé conforme à la revendication 1, 2 ou 3, dans lequel le composé est le 5-hydroxy-2-cinnamoyl-2,4,6,7-tétraméthyl-2,3-dihydrobenzofuranne.

12. Procédé conforme à la revendication 1, 2 ou 3, dans lequel le composé est le 5-hydroxy-2-phényléthyl-2,4,6,7-tétraméthyl-2,3-dihydrobenzofuranne.

13. Procédé conforme à la revendication 1, 2 ou 3, dans lequel le composé est le 5-hydroxy-2-(1-(Z)-pentényl)-2,4,6,7-tétraméthyl-2,3-dihydrobenzofuranne.

14. Utilisation d'un composé défini dans la revendication 2 ou d'un sel d'un tel composé, ou du 5-hydroxy-2,4,6,7-tétraméthyl-2-(3',7',11'-triméthyl-dodécyl)-2,3-dihydrobenzofuranne, comme composant dans la préparation d'un médicament inhibant la 5-lipoxygénase et/ou la 12-lipoxygénase.